(19) Europäisches Patentamt
European Patent Office
Office européen des brevets

(11) Veröffentlichungsnummer : **0 047 870**
**B1**

(12) # EUROPÄISCHE PATENTSCHRIFT

(45) Veröffentlichungstag der Patentschrift : **05.06.85**

(51) Int. Cl.⁴ : **A 61 B 5/00**

(21) Anmeldenummer : **81106386.6**

(22) Anmeldetag : **17.08.81**

(54) **Geräteanordnung, bestehend aus einer vorgebbaren Zahl von Überwachungsgeräten und wenigstens einer Gerätezentrale.**

(30) Priorität : **12.09.80 US 186710**

(43) Veröffentlichungstag der Anmeldung : **24.03.82 Patentblatt 82/12**

(45) Bekanntmachung des Hinweises auf die Patenterteilung : **05.06.85 Patentblatt 85/23**

(84) Benannte Vertragsstaaten : **BE CH DE FR GB IT LI NL**

(56) Entgegenhaltungen :
**DE-A- 2 146 311**
**US-A- 3 814 082**
**US-A- 4 051 522**
**US-A- 4 216 462**

(73) Patentinhaber : **Siemens Aktiengesellschaft**
**Berlin und München Wittelsbacherplatz 2**
**D-8000 München 2 (DE)**

(72) Erfinder : **Neumann, Leopold**
**9 Woodpark Circle**
**Lexington Massachusetts 02173 (US)**
Erfinder : **Kline II, Richard B.**
**25 Dean Street**
**Stoneham Massachusetts 02180 (US)**
Erfinder : **Scholz, Wolfgang**
**4 Berrywood Lane**
**Beverly Massachusetts 01915 (US)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 047 870 B1

**Beschreibung**

Die Erfindung bezieht sich auf eine Geräteanordnung mit einer vorgegebenen Zahl von Überwachungsgeräten und wenigstens einer Gerätezentrale, die ein zentrales Darstellungsgerät mit diesem zugeordneten Zwischenspeicher und einem zentralen Multiplexer enthält, wobei jedes Überwachungsgerät wenigstens einen Signalgeber umfaßt, dessen Signale an einem dem betreffenden Überwachungsgerät eigenen Darstellungsgerät als gerätespezifisches Bild darstellbar sind, und wobei die Gerätezentrale mit den Signalgebern der Überwachungsgeräte durch eine Anwähleinrichtung verbindbar ist derart, daß die Gerätezentrale von dem Signalgeber eines beliebig angewählten Überwachungsgerätes Signale zur Darstellung auf dem zentralen Darstellungsgerät übernimmt.

Geräteanordnungen dieser Art können auf den unterschiedlichsten Meßgebieten eingesetzt werden. Ein bevorzugtes Anwendungsgebiet ist jedoch das der Elektromedizin. Hier werden vom Körper eines Patienten die unterschiedlichsten physiologischen Signale abgenommen und zum Zwecke einer Überwachung auf kritische Zustände z. B. am Bildschirm einer Kathodenstrahlröhre oder auf dem Papier eines Schreibers oder an ähnlichen Darstellungsgeräten dargestellt. Bei den abgenommenen und darzustellenden Signalen handelt es sich um so unterschiedliche Signale wie z. B. Elektrokardiogramme (EKG), Blutdrucksignale, Atemsignale, $CO_2$-Signale (Gehalt von Kohlendioxid im Blut oder Atemgas), Temperatursignale, etc.

Besondere Eigenschaft solcher Geräteanordnungen ist, daß an den Darstellungsgeräten der einzelnen Überwachungsgeräte die anfallenden Signale mit den unterschiedlichsten Aufzeichnungsgeschwindigkeiten dargestellt werden können. Damit besteht zwar die Möglichkeit der Übernahme eines kompletten Signalbildes von einem einzelnen Überwachungsgerät zum zentralen Darstellungsgerät der Gerätezentrale. Eine Überspielung einzelner Signalspuren, die aus Signalbildern unterschiedlicher Überwachungsgeräte stammen, als Mischbild verbietet sich jedoch im allgemeinen, wenn die zu überspielenden unterschiedlichen Signalspuren auf unterschiedlichen Geschwindigkeiten laufen.

Eine Geräteanordnung der eingangs genannten Art ist aus der US-A-4 216 462 bekannt. Bei dieser Geräteanordnung ist zwischen den Signalgebern der Überwachungsgeräte und dem zentralen Darstellungsgerät der Gerätezentrale ein Multiplexer eingeschaltet. Dem Darstellungsgerät ist auch ein Zwischenspeicher zugeordnet. Der Multiplexer und der Zwischenspeicher (Bildwiederholungsspeicher) dienen nun aber keineswegs dem Aufbau von Mischbildern. Sinn und Zweck dieser Bauglieder ist es vielmehr, im Alarmfall sozusagen die Historie des kritischen Zustandes des Patienten für die bildliche Darstellung bereitzuhalten. Mit anderen Worten, es soll im Alarmfall das dargestellt werden, was eine vorgegebene Zeit (z. B. 6 Sekunden) vor Auftreten des Alarms an physiologischen Parametern vorlag.

Aufgabe der Erfindung ist es, eine Geräteanordnung der eingangs genannten Art anzugeben, die den Aufbau von Mischbildern an dem zentralen Darstellungsgerät jederzeit erlaubt, d. h. unabhängig davon, ob an den einzelnen Überwachungsgeräten Signalspuren gerade mit unterschiedlicher Geschwindigkeit dargestellt werden oder nicht.

Diese Aufgabe wird erfindungsgemäß dadurch gelöst, daß zwischen den Signalgebern der Überwachungsgeräte und dem zentralen Darstellungsgerät der Gerätezentrale der zentrale Multiplexer eingeschaltet ist, der für das Ausgangssignal eines jeden Signalgebers eines Überwachungsgerätes einen separaten Signaleingang besitzt und der eine erste und eine zweite Zahl von Ausgängen aufweist, von denen die erste Zahl an Ausgängen direkt in Richtung auf das zentrale Darstellungsgerät geht, während die zweite Zahl an Ausgängen zum Eingang des Zwischenspeichers geht, dessen Signalausgang in Richtung auf das zentrale Darstellungsgerät führt, und daß der zentrale Multiplexer von einer Steuereinheit so gesteuert ist, daß er der ersten Zahl von Ausgängen im Zeitmultiplexer jeweils nur solche Signaldaten zuführt, die vom Signalgeber eines einzelnen ausgewählten Überwachungsgerätes stammen, während er der zweiten Zahl von Ausgängen nur die jeweils ältesten Signaldaten aller Signalgeber zuführt, wobei die jeweils ältesten Signaldaten im Zwischenspeicher gespeichert werden und dort in beliebigen Signalkombinationen zur Aufzeichnung als Mischbild auf dem zentralen Darstellungsgerät abrufbar sind.

An einem einzelnen Überwachungsgerät ist die Darstellungsgeschwindigkeit für alle Signale eines Signalbildes gleich. Das Zentralgerät kann also jederzeit einerseits das komplette Signalbild eines beliebigen Überwachungsgerätes zur Direktdarstellung auf dem zentralen Darstellungsgerät übernehmen. Andererseits kann auch jederzeit aus den im Zwischenspeicher gespeicherten ältesten Signalwerten ein Mischbild komponiert und zur Darstellung am zentralen Darstellungsgerät abgerufen werden. Die jeweils ältesten Signalwerte stammen zwar aus Signalbildern, die an unterschiedlichen Überwachungsgeräten möglicherweise mit unterschiedlichen Darstellungsgeschwindigkeiten anfallen; sie werden demnach durchaus auch mit sehr unterschiedlichen Geschwindigkeiten in den Zwischenspeicher eingespeichert. Das Auslesen zum zentralen Darstellungsgerät geschieht jedoch durchwegs mit einer für alle gespeicherten Signalwerte gleichen Auslesegeschwindigkeit. Der Zwischenspeicher wirkt also als eine Art Geschwindigkeitspuffer, der primär unterschiedliche Darstellungsgeschwindigkeiten über den Umweg einer verzögerten Darstellung einander angleicht und somit den Aufbau beliebiger Mischbilder erlaubt.

In vorteilhafter Ausgestaltung der Erfindung ist der zentrale Zwischenspeicher ein Bildwiederholungsspeicher, der von den ebenfalls als Bildwiederholungsspeichern ausgebildeten Signalgebern der einzelnen Überwachungsgeräte immer solche Signalwerte erhält, die gerade als älteste Werte (delayed data) durch neueste Werte ersetzt wurden. Als Bildwiederholungsspeicher können dabei z. B. dynamische RAM-Speicher oder auch dynamische Schieberregister od. dgl. eingesetzt werden.

Weitere Vorteile und Einzelheiten der Erfindung ergeben sich aus der nachfolgenden Beschreibung eines Ausführungsbeispiels anhand der Zeichnung und in Verbindung mit den weiteren Ansprüchen.

Es zeigen :

Figur 1 ein Ablaufdiagramm für drei Koordinaten x, y, z, einer Signalkombination,

Figur 2 eine Aufzeichnung von Analogsignalen und Zeichensignalen auf dem Bildschirm eines x, y, z-Oszilloskops,

Figur 3 eine Aufzeichnung ähnlicher Art mit Grenzwerten,

Figur 4 den Zeitablauf der Signalfolge während einer x-Ablenkung,

Figur 5 die Anwendung der Erfindung auf ein elektromedizinisches Überwachungsgerät im Prinzipschaltbild,

Figur 6 einen Ausschnitt aus dem Prinzipschaltbild der Figur 5 in detailierter Darstellung,

Figur 7 eine Anwendung auf eine Anordnung von Einzelgeräten in Reihenschaltung ohne gemeinsame zusätzliche Zentrale,

Figur 8 die Anwendung gemäß der Erfindung auf eine Anordnung von Einzelgeräten in Sternschaltung zu einer gemeinsamen zusätzlichen Zentrale,

Figur 9 einen zentralen Multiplexer als Bestandteil der Zentrale der Sternschaltung gemäß Figur 8,

Figur 10 eine Steuereinrichtung für Daten- und Adress-Steuerung zwischen zentralem Multiplexer und einem zentralen Bildwiederholungsspeicher als Bestandteil der Zentrale der Sternschaltung gemäß Figur 8,

Figur 11 ein Diagramm für digitale Signalübertragung in der Rückführphase (retrace time) der x-Ablenkung (als weitere Ausgestaltung des Diagramms der Figur 4),

Figur 12 den mechanischen Prinzipaufbau eines elektromedizinischen Überwachungsgerätes mit vier Einschubmodulen, so wie es in Verbindung mit der Erfindung bevorzugt als Bedside — Gerät eingesetzt wird, in perspektivischer Ansicht,

Figur 13 den prinzipiellen inneren Schaltungsaufbau eines Einschubmoduls (hier insbesondere kombinierter EKG/Blutdruck-Modul),

Figur 14 den prinzipiellen inneren Schaltungsaufbau des Kontroll- und Ankoppelboards, wie es den Einschüben zur Signal- und Energieankopplung auf der Seite des Gerätes gegenübersteht,

Figur 15 ein Pulsdiagramm der wichtigsten Pulsfolgen, so wie sie zwischen Einschubmodulen und Gerät in der einen und in der anderen Richtung übertragen werden,

Figur 16 das Zusammenspiel zwischen vorverarbeitendem Mikroprozessor (preprocessor), Hauptspeicher (shared memory) und zentralem Mikroprozessor (central processor) eines Gerätes gemäß Figur 5 im detaillierten Prinzipschaltbild,

Figur 17 die Signal- und Zeichendarstellung der Figur 2 mit wanderndem Vertikalbalken als Grenze zwischen neuer und alter Signalinformation bei fixed mode-Betrieb,

Figur 18 das Prinzipschaltbild zur Erzeugung eines Vertikalbalkens gemäß Figur 17.

In der Figur 1 sind die drei Koordinateninformationen für jede Signalperiode in Abhängigkeit von der Zeit t übereinander als x(t), y(t), z(t) dargestellt. Jeder Koordinatenverlauf ist in Zeitabschnitte I', I, II, III, IV (durch Ordinate und strichpunktierte Linien zu den Zeitpunkten $t_0$, $t_1$, $t_2$, $t_3$, $t_4$, $t_5$ angedeutet) unterteilt. Der Abschnitt I' dient zaur Übertragung eines später noch näher beschriebenen y-Konfigurationswortes im Anschluß an die Auslösung einer x-Ablenkung. Der Abschnitt IV ist die später ebenfalls noch näher erläuterte Rückführphase (retrace time) der z-Ablenkung eines Elektronenstrahles eines x, y, z-Oszilloskops im jeweiligen Gerät. Die Abschnitte I, II, II sind die Hauptabschnitte zur Aufzeichnung von alphanumerischen Zeichen und von Analogsignalen. Sie werden im folgenden auch als Felder (fields) für Analog- oder Zeichendarstellung bezeichnet. Gemäß dem Diagramm der Figur 1 beginnt also zum Zeitpunkt $t_0$ die z-Ablenkung mit der Rampenspannung $X_R$. Nach Übertragung des y-Konfigurationswortes im Abschnitt I' folgt zum Zeitpunkt $t_1$ ein schnelles y-Raster $Y_R$. Dieses Raster ist Grundlage für eine Zeichendarstellung. Die beiden Größen x und y sind im Feld I als Rampenspannungen systemeigene konstante Größen, die im Gerät selbst erzeugt werden können. Eine selbständige Übertragung solcher Raster zum einzelnen Gerät über eine gemeinsame Signalleitung (Signal Bus) erübrigt sich also. Es brauchen lediglich die zur Auslösung der Rampenspannungen $X_R$ und $Y_R$ nötigen Auslösesignale in Form eines x-Synchronisiersignales bzw. eines y-Konfigurationssignales weitergereicht werden. Das Vorliegen zweier systemkonstanter Koordinaten x, y im Feld I kann jedoch dazu ausgenutzt werden, über eine gemeinsame Signalleitung (Signal Bus) Adress-Signale für einen Zeichengenerator zu übertragen, der sich im jeweiligen Gerät befindet. Aufgrund dieser Adress-Signale, die in Digitalform übertragen werden, bildet der Zeichengenerator im jeweils angesteuerten Gerät Helltastsignale, die in zeitlicher Synchronität mit dem gleichzeitig im Gerät erzeugten raschen y-Zeichenraster am Bildschirm eines x, y, z-Oszilloskops zu den erwünschten alphanumerischen Zeichen zusammengesetzt werden. Die im Informationsabschnitt I zu übertragenden Adress-Signale für den Zeichengenerator sind in der Figur 1 für die Koordinate z als digitale Signale CA (Character Addresses) angedeutet.

Signifikante Eigenschaft des Abschnittes I (Feld I) ist also, daß die Koordinaten x und y systemeigen konstant sind, während die Koordinate z systemfremd und variabel ist. Da jedoch zwei Koordinatengrößen konstante Größen sind, kann die dritte variable Größe z über die gemeinsame Signalleitung zum jeweiligen Gerät übertragen werden.

Im Informationsabschnitt II (oder Feld II) des Diagramms der Figur 1 ergibt sich hingegen geänderte Situation. Neben der dauernd systemkonstanten x-Koordinate ist jetzt die y-Koordinate als Übertragungsabschnitt eines Analogsignales A (z. B. EKG) systemfremd und variabel. Damit handelt es sich also bei der Koordinate y im Falle des Feldes II nicht um eine systemeigene konstante Größe ; um diese Größe A also übertragen zu können, muß neben der x-Koordinate im Feld II auch die z-Koordinate systemeigen und konstant sein. Dies ist sie auch, denn gemäß dem Diagramm der Figur 1 zeigt die z-Koordinate im Informationsabschnitt II konstanten Spannungsverlauf.

Betreffend Feld III ergeben sich zwei Möglichkeiten : Hier kann einerseits bei Bedarf jener Signalverlauf, wie er sich für Feld II ergab, für alle drei Koordinaten x, y, z unverändert beibehalten werden. Dementsprechend würde also im Feld III bei konstanter Spannung der z-Koordinate die Analoggröße A im Signalweg als y-Komponente fortgesetzt übertragen werden.

Andererseits kann jedoch auch auf Abbildung entsprechend dem Muster des Feldes I rückgeschaltet werden. In diesem Falle wird also im Feld III hinsichtlich der y-Koordinate wieder ein rasches y-Ablenkraster $Y_R$ für Zeichendarstellung erzeugt und im Signalweg zu den Geräten werden während dieser Zeitdauer als z-Komponente also wieder Adress-Signale CA zur korrekten Adressierung im Zeichengenerator übertragen.

Die Darstellung der Figur 1 ist lediglich beispielhaft. Es ist selbstverständlich, daß die einzelnen Felder I bis III beliebig variabel aneinandergereiht werden können. Ebensogut kann die Zahl sämtlicher Felder von drei abweichend vorgegeben werden. Dabei können in unterschiedlichen Feldern wahlweise Analogsignale zusammen mit Zeichen dargestellt werden. Es können aber in einer einzigen Spur auch nur Analogsignale oder nur Zeichen abgebildet werden. Wie eingangs schon angedeutet, kann der den Hauptfeldern I bis III vorangehende Abschnitt I' allein zur Übertragung eines y-Konfigurationssignales verwendet werden. Es ist wieder selbstverständlich, daß zusätzlich zum Konfigurationssignal in diesem Abschnitt auch noch weitere beliebig gestaltete Befehlssignale übertragen werden können. Derartige zusätzliche Signale können aber auch, wie eingangs ebenfalls schon angedeutet, in jene Phase gelegt werden (z. B. Felder I und/oder III), in denen zur Zeichendarstellung rasche y-Raster erzeugt werden. Die zusätzlichen Befehlssignale werden dann zusammen mit den digitalen Adress-Signalen CA in zweckmäßig gemischter Form über den gemeinsamen Signalweg übertragen. Darüber hinaus können derartige zusätzliche Signale auch in der Phase IV, d. h., in der Rückführphase des Elektronenstrahls nach einer x-Ablenkung, übertragen werden.

Die Zusammensetzung der einzelnen Informationsabschnitte von Analogsignalen und Zeichen zu einem Gesamtbild am Bildschirm eines x, y, z-Oszilloskops zeigen beispielsweise die Figuren 2 und 3. Hier ist der Bildschirm des Oszilloskops mit B bezeichnet. Man sieht sofort, daß sowohl in Figur 2 als auch in Figur 3 im Feld I am Bildschirm B alphanumerische Zeichen dargestellt werden. Im Feld II werden Abschnitte eines Analogsignales A aufgezeichnet. In Figur 2 wird im Feld III die Aufzeichnung dieses Analogsignales fortgesetzt. In Figur 3 werden hingegen in Feld III erneut alphanumerische Zeichen in Form von Grenzwerten (limits) eingeblendet.

Bei den Oszilloskopen der Figuren 2 und 3 erfolgt die Strahlablenkung des Elektronenstrahls zur Signalaufzeichnung in Draufsicht auf den Bildschirm B von rechts nach links. Auf der rechten Seite des Bildschirmes B erscheinen im Feld I also immer zuerst die alphanumerischen Zeichen. Daran schließt sich das Feld II mit der Darstellung eines Analogsignales an. Das Feld III kann dann entweder zur Fortsetzung der Aufzeichnung des Analogsignales (Figur 2) oder zur erneuten Einblendung von alphanumerischen Zeichen (Figur 3) genutzt werden.

Aus den Figuren 2 und 3 ist auch zu ersehen, daß es sich bei den dargestellten Oszilloskopen beispielsweise um Vierkanal-Oszilloskope handelt. Diese Oszilloskope arbeiten also in der Weise, daß in zeitlicher Aufeinanderfolge in untereinander geschichteten Kanälen insgesamt bis zu vier unterschiedliche Analogsignale mit entsprechend zugeordneten Zeichen dargestellt werden können. In jedem Zeichenfeld I und/oder III können dabei durch geeignete geschichtete Adressenansteuerung im Zeichengenerator pro Kanal ein oder auch mehrere Zeichen (z. B. wie dargestellt bis zu zwei Zeichen) gleichzeitig einer einzelnen Signalkurve zugeordnet werden. In den vorliegenden Ausführungsformen der Figuren 2 und 3 sind also auf dem Bildschirm des jeweiligen Vierkanal-Oszilloskops im Kanal 1 z. B. das EKG eines Patienten zusammen mit Zeichen abgebildet, welche Zeichen für beide Figuren in Spalte I z. B. den Wert der Herzfrequenz und für Figur 3 in Spalte III speziell auch noch einen oberen und einen unteren Grenzwert der Herzfrequenz angeben. In den Kanälen 2 und 3 werden hingegen beispielsweise die Verläufe von zwei Blutdruckkurven dargestellt. Die in den Feldern I in beiden Figuren eingeblendeten Zeichen sind Wertangaben für den jeweiligen Blutdruckwert von Systole, Diastole oder Mitteldruck. Die in Figur 3 in Feld III abgebildeten Zeichen sind wieder Grenzwertangaben für die Blutdruckwerte. Der Kanal 4 zeigt schließlich den Verlauf des $CO_2$-Gehaltes im Atemgas des Patienten mit eingeblendeten Größen- bzw. Grenzwertangaben für den $CO_2$-Gehalt. Den aufzuzeichnenden Analogsignalen sind also im Sichtbild wahlweise nur reine Größenangaben oder zusätzlich zu den reinen Größenangaben auch noch Grenzwertangaben über maximal oder minimal zulässige Grenzwerte der physiologischen Meßsignale in Form alphanumerischen Zeichen zuzuordnen. Durch Beigabe von Grenzwertinformationen können Grenzwertüberschreitungen und damit auch kritische Entwicklungen einer physiologischen Meßgröße sofort erkannt werden. Kritische Grenzwertüberschreitungen werden im vorliegenden Falle aber auch noch durch Abgabe eines entsprechenden Alarmsignales (insbesondere akustischer und/oder optischer Natur) angezeigt. Falls im Grundgerät der Bildinhalt eines Trendspeichers dargestellt ist, sind die in den Figuren 2 und 3 abgebildeten Analogkurven entsprechend durch Trendkurven ersetzt.

Für die Qualität des abzubildenden Systems ist es wesentlich, daß die Bilddarstellung aller abzubildenden Signale flackerfrei erfolgt. Um Flackerfreiheit der Bildaufzeichnung gewährleisten zu können, ist eine 50 Hz-Bildwechselfrequenz anzustreben. Die Gesamtzeit der Strahlablenkung über den Bildschirm des jeweiligen Oszilloskops ist demfolge 20 ms. Wird eine Aufzeichnung in vier Kanälen gewünscht, so darf die gesamte Ablenkzeit inklusive Rücklauf pro Kanal also höchstens 5 ms betragen.

Einen in diesem Sinne angepaßten Zeitplan zur Signalübertragung zeigt die Figur 4. Im Zeitplan der Figur 4 ist die Gesamtdauer T einer x-Ablenkung mit T = 4.880 µs angegeben. Diese Zeitdauer T ist unterteilt in insgesamt fünf Zeitabschnitte I', I, II, III, IV. Wie vorstehend schon erwähnt, wird zu Beginn jeweils ein Synchronisierimpuls SP (3 Volt, 3 µs) gesetzt, der z. B. mit seiner Rückflanke zum Zeitpunkt $t_0$ den Beginn einer x-Ablenkung festlegt. Die Steuerung sämtlicher betroffener Geräte durch den Synchronisierimpuls ist so, daß immer der jeweils zuerst auftretende Synchronisierimpuls eines einzelnen Gerätes die x-Ablenkung sämtlicher anderer Geräte zeitlich miteinander synchronisiert, es sei denn, es wird von vornherein durch eine externe Zentrale ein Synchronisierimpuls allen anderen Geräten aufgedrückt. Auf den Synchronisierimpuls SP folgt nun zuerst das Zeitintervall I', das im Vorliegenden Falle 80 µs lang ist. Während der Dauer dieses Zeitintervalls I', das zum Zeitpunkt $t_1$ endet, wird an sämtliche anzusteuernden Geräte der Gerätekonfiguration ein Ablenk-Konfigurations-Signal (8 bit-sweep configuration word) als Digital-Signal übermittelt, das als Hinweissignal dafür dient, wie Beginn und/oder Ende sowie Art und Ort der momentanen y-Darstellung am Bildschirm eines gerade eingeschalteten Oszilloskops im Vergleich mit der zu übertragenden Signaldarstellung eines anderen Gerätes zu wählen sind. Das Ablenk-Konfigurations-Signal kann als 8 bit-Wort in den Stufen BIT 0 bis BIT 7 für vorliegendes Ausführungsbeispiel in der Anwendung auf ein Vierkanal-Oszilloskop wie folgt definiert sein :

| BITS | | BESCHREIBUNG |
|---|---|---|
| BIT 0 : | | Feld III Steuerung<br>Null = Analogsignaldarstellung (+ 0.75 V in CV)<br>Eins = Zeichendarstellung (− 1.0 V in CV) |
| BIT 1 : BOT 0<br>BIT 2 : BOT 1 | | Diese BIT's kontrollieren die vertikale Position der Grundlinie (BOT) der Spur für die Darstellung analoger Parameter gemäß folgender Aufstellung : |
| BOT 1 | BOT 0 | |
| 0 | 0 | Untere Position |
| 0 | 1 | Mittlere untere Position |
| 1 | 0 | Mittlere Obere Position |
| 1 | 1 | Obere Position |
| BIT 3 : RT 0<br>BIT 4 : RT 1 | | Diese BIT's kontrollieren die vertikale Position der Zeichendarstellung und die Darstellung der Rückspur des Elektronenstrahls nach x-Auslenkung. Die definieren auch die jeweilige Spur und sind wie folgt kodiert : |
| RT 1 | RT 0 | |
| 0 | 0 | Kanal 4 Unten |
| 0 | 1 | Kanal 3 Mittel Unten |
| 1 | 0 | Kanal 2 Mittel Oben |
| 1 | 1 | Kanal 1 Oben |
| BIT 5 : G 0<br>BIT 6 : G 1 | | Diese BIT's kontrollieren die Y-Ablenkverstärkung für analoge Signalformen gemäß folgender Aufstellung : |
| G 1 | G 0 | |
| 0 | 0 | Verstärkung = 3 |
| 0 | 1. | Verstärkung = 2 |
| 1 | 0 | Verstärkung = 1 normal |
| 1 | 1 | Verstärkung = 0 Austastung |
| BIT 7 : | | Intensität der Rückspur. Die Rückspur in der Position, wie sie durch RT 0, RT 1 definiert ist, wird aufgeblendet, wenn das BIT eine EINS ist. |

Im Anschluß an den Abschnitt I' folgt das erste Feld I. Die Dauer dieses Feldes ist 1.280 µs. Zum Zeitpunkt $t_2$ folgt auf das Feld I das Feld II, das wieder 1.280 µs lang ist. An das Feld II schließt sich zum Zeitpunkt $t_3$ das Feld III an, das ebenfalls 1.280 µs andauert. Die x-Ablenkung wird anschließend abgeschlossen durch den Abschnitt IV, der 960 µs lang ist und der die Rückführzeit für den Elektronenstrahl festlegt. In der Signaldarstellung der Figuren 2 und 3 wird der rückgeführte Elektronenstrahl durch Spezialwahl im Konfigurationswort jeweils als dünne Spur R in die Bildaufzeichnung mit eingeblendet. Die Rückspur R signalisiert den Verlauf der Null-Linie in jedem Kanal. Die Rückführphase IV für den Elektronenstrahl kann zur Übertragung von beliebigen zusätzlichen Befehlssignalen ausgenutzt werden. Ebensogut können zyklisch umlaufende Speicherwerte der Analogsignale während der retrace time aus dem Bildwiederholungsspeicher als jeweils älteste Werte entfernt und beispielsweise einem mitlaufenden Schreiber, Rekorder oder dergleichen als sogenannte verzögerte Daten (Delayed Data) zugeführt werden.

Darüber hinaus können bei Bedarf während der Phase IV auch Daten in beliebiger Weise in Speichern umgeschichtet bzw. auch endgültig gelöscht werden. Das Ende der Retrace Phase IV ist zugleich der Beginn einer neuen Ablenkperiode. Es wird zum Zeitpunkt $t_5$ also jeweils ein neuer Synchronisierimpuls SP erzeugt, der den Elektronenstrahl nach Umschaltung auf den jeweils nächstfolgenden Kanal eines Oszilloskops mit seiner Rückflanke zum Zeitpunkt $t_6 = t_0$ erneut zur x-Ablenkung triggert. Die Triggerung erfolgt immer in der Weise, daß der Elektronenstrahl des Oszilloskops unabhängig davon, wo er sich in der Rückphase gerade am Bildschirm befindet, sofort zu seiner Ausgangsstellung in der rechten äußeren Randposition des Bildschirms zurückgezogen wird. Die sich aufgrund der abrupten Unterbrechung der Rückführphase ergebende momentane Instabilität wird durch das sich an den Synchronimpuls anschließende Zeitintervall I' mit Übermittlung eines y-Konfigurationssignales, das ja selbst am Bildschirm nicht abgebildet wird, überbrückt.

Die Erfindung findet ihre Anwendung insbesondere im Medizinbereich bei der Abnahme physiologischer Signale, wie insbesondere EKG, Blutdruck, Temperatur $CO_2$ etc. am Körper des Patienten. Ein einzelnes Gerät dieser Art ist beispielsweise in der Figur 5 im Prinzipschaltbild dargestellt. Dieses Gerät umfaßt insgesamt vier Einschubmodule 1 bis 4 zur Aufnahme und Weiterleitung abgenommener physiologischer Signale zu einer Modul/Koppel- und Steuereinheit 5. Diese Einheit 5 ist mit jedem einzelnen Einschubmodul 1 bis 4 energiemäßig über je ein Paar Energiekoppelspulen 6 und 7 zur Übertragung der zum Betrieb der Einschubmodule benötigten Energie von der Einheit 5 zum Modul 1 bis 4 gekoppelt. Die Übertragung der physiologischen Signale oder sonstigen empfangenen Signale von einem Einschubmodul zur Einheit 5 geschieht mittels Optokoppler 8, 9. Mit 8 ist dabei eine Sendediode, insbesondere Lumineszenzdiode für Infrarotlicht, bezeichnet. Die Kennziffer 7 deutet einen Lichtempfänger, insbesondere Fotodiode, an. Zur Übertragung von Signalen, z. B. Schaltsignalen, A/D-Taktsignalen oder sonstigen Steuersignalen von der Einheit 5 zu einem Einschubmodul 1 bis 4 ist ein weiterer Optokoppler 10, 11 vorhanden. Bei diesem Optokoppler befindet sich also eine Sendediode 10 an der Einheit 5 und eine Empfangsfotodiode am jeweiligen Modul 1 bis 4. Nähere Einzelheiten zum inneren prinzipiellen Schaltungsaufbau eines Moduls 1 bis 4 sowie der Modul/Koppel- und Steuereinheit 5 können der weiter unten folgenden Beschreibung zu den Figuren 13 bis 15 entnommen werden.

In praktischer Ausbildung der Anordnung (siehe hierzu auch die Figur 12) umfaßt das Gerät im Gerätegehäuse eine Einbuchtung, in die die Einschubmodule 1 bis 4 etagenmäßig übereinander einschiebbar sind. Die Einbuchtung umfaßt eine Rückwand, an der die Einheit 5 mit den ersten Halbteilen der jeweiligen Energiekoppelstelle bzw. des jeweiligen Optokopplers montiert sind. Das jeweils andere Halbteil der Energiekoppelstelle bzw. des jeweiligen Optokopplers ist, wie in der Figur 5 angedeutet, an der Rückseite des jeweiligen Einschubmoduls 1 bis 4 angeordnet. Wird das Einschubmodul also in der Einbuchtung des Gerätegehäuses in seine Endstellung eingeschoben, so fügen sich die von ihm getragenen Halbteile jeder Kopplungsstelle mit den Halbteilen der Rückwand zu einer kompletten galvanisch trennenden Koppelstelle für Energie- und Signalübertragung zusammen. Die Einheit ist betriebsbereit.

Die von der Einheit 5 aufgenommenen physiologischen oder sonstigen Signale werden nun über den Datenpfeil 12 einem Mikroprozessor 13 für Datenverarbeitung (Preprocessor), insbesondere Datenskalierung und Datenwichtung, zugeleitet. Wie später in der Beschreibung zu Figur 16 noch näher erläutert wird, transferiert dieser Mikroprozessor die vorverarbeiteten Daten in einen zentralen Mikroprozessor 14 via eines gemeinsamen adressierbaren Teils eines Hauptspeichers 15 (shared memory) unter Zuhilfenahme einer speziellen Hardware, die den direkten Zugriff DMA zu Speicherplätzen ermöglicht (DMA = Direct Memory Access). Zusätzlich beinhaltet der vorverarbeitende Mikroprozessor 13 auch noch einen On-Chip UART 16 (UART = Universal Asynchronous Receiver Transmitter) zur Kommunikation mit einer Zentrale 17. Der Mikroprozessor 13 für die Vorverarbeitung kann außerdem gemäß Datenpfeil 18 Signaldaten, wie Taktsignale, Konfigurationssignale oder dergleichen, über die Einheit 5 unter Einschaltung des Signalweges über die Optokoppler 10, 11 zu den einzelnen Einschubmodulen 1 bis 4 übersenden. Der Speicher 15 ist normalerweise dem zentralen Mikroprozessor 14 fest zugeordnet (Datenpfeil 19). Der vorverarbeitende Mikroprozessor 13 hingegen ist abgeschaltet. Lediglich bei DMA-Anforderung seitens des vorverarbeitenden Prozessors (Leitung 20) trennt sich der zentrale Mikroprozessor 14 vom Speicher 15 und der vorverarbeitende Prozessor 13 übernimmt die Kontrolle über den Speicher (Datenpfeil 21).

Der zentrale Mikroprozessor 14 ist das Herzstück des Gerätes. Von ihm werden über eine Datenleitung 22 die anfallenden Signaldaten über ein zentrales Schieberegister 23 in einen dynamischen RAM-Speicher 24 unter der Kontrolle eines Adress-Rechners 25, der vom zentralen Mikroprozessor 14 über eine Adressleitung 26 Adressdaten erhält, eingelesen. Das zentrale Schieberegister 23 übernimmt dabei alle anfallenden Daten in paralleler Formation und liest sie seriell wieder aus. Die Taktfolge des Ein- und Auslesens von Daten wird von einem zentralen Taktgeber 27 vorgegeben. Die vom RAM-Speicher 24 zum Oszilloskop 28 zur Aufzeichnung weiterzuleitenden Daten werden in einer Verarbeitungsvorrichtung 29 vorverarbeitet und dann auf das Oszilloskop gegeben. Die Vorrichtung 29 beinhaltet dabei alle Bauelemente, die zur Erzeugung bzw. zur Weiterleitung der erfindungsgemäßen Signalfolge (Composite Video Signal) gemäß der Figur 1 notwendig sind. Mittels der Vorrichtung 29 werden also Signale und Zeichen entsprechend den Figuren 1 bis 4 aufbereitet und dem geräteeigenen Monitor 28 zugeleitet. Gleichzeitig werden sämtliche in diesem Sinne aufbereiteten Signale auch über einen Ausgang 30 (Composite Video Output) auf eine für sämtliche Geräte einer beliebigen Gerätekonfiguration gemeinsame Übertragungsleitung (Composite Video Bus) gegeben. Darüber hinaus umfaßt die Vorrichtung 29 aber auch noch einen entsprechenden Eingang 31 (Composite Video Input) über den das Gerät ebenfalls an der gemeinsamen Verbindungsleitung für alle Geräte anschaltbar ist. Von dort können also im Bedarfsfall, z. B. im Falle eines Alarmes, unter Abschaltung des eigenen Datenflusses zum Oszilloskop, von einem beliebigen anderen Gerät, insbesondere jenem des Alarmfalles, Composite Video Signale aus dem gemeinsamen Übertragungsweg zur Aufzeichnung auf dem geräteeigenen Oszilloskop 28 übernommen werden. Der Alarm wird von einem am zentralen Mikroprozessor 14 angeschalteten Alarmgeber 32 erzeugt und z. B. in Pfeilrichtung 33 auf einen Alarm Bus gegeben. Zur Eingabe besonderer Befehlsdaten in den zentralen Prozessor 14 dient ein Tastenfeld 34, z. B. an der Frontplatte des jeweiligen Gerätes, mit Sensor 35 für die richtige Eingabe. Zur Anzeige eingegebener oder während der Signalverarbeitung anfallender Daten dient eine LED-Anzeige 36 (Leuchtdioden) mit Treiberstufe 37. Ein Lautsprecher 38 mit Verstärker 39 dient zur akustischen Signalanzeige, z. B. für Alarm, für Tastklicks der Tasten eines Tastenfeldes bzw. Takt für anfallende QRS-Pulse oder auch Anzeige für unterbrochenen Programmablauf des zentralen Prozessors. Das Bauteil 40 ist ein ROM- und RAM-Zusatzspeicher für den zentralen Mikroprozessor.

Die Vorrichtung zur Erzeugung und Weiterleitung bzw. auch des Empfangs von Composite Video Signalen ist als Bestandteil z. B. eines Gerätes gemäß der Figur 5 im Prinzipschaltbild der Figur 6 in Detail dargestellt. Demgemäß gabelt sich der Ausgang des zentralen Schieberegisters 23 in Richtung Signalverarbeitungsvorrichtung 29 in wenigstens drei Signalpfade 41, 42 und 43. In jedem dieser Signalpfade ist ein spezielles Signalverarbeitungsglied eingeschaltet. So befindet sich im Signalpfad 41 ein Decoder 44 für das y-Konfigurationswort. Im Signalpfad 42 liegen hingegen der Zeichengenerator 45 für die Zeichenbildung (lokales ROM für 2 × 7 Character Display im 8 bit code) und der Ramp-Generator 46 zur Erzeugung des schnellen y-Ablenkrasters (y-Rampenspannung $Y_R$). Im Signalpfad 43 ist schließlich der Digital-Analog-Umsetzer 47 zur Regenerierung der Analogsignale A eingeschaltet. Die Ausgänge des Decoders 44 für das y-Konfigurationssignal, des Generators 46 für das y-Ablenkraster und des Digital-Analog-Umsetzers 47 führen zu einer Einrichtung 48 für die Verarbeitung aller eingehenden Signale zum vertikalen Ablenksignal y für das nachgeschaltete Oszilloskop 28. Der Zeichengenerator 45 geht mit einem weiteren Ausgang auf die Helltasteinrichtung 49 des Oszilloskops, die die zum Zeichenaufbau nötigen Helltastimpulse liefert. Zur x-Ablenkung ist ein x-Rampgenerator 50 vorhanden, der direkt vom internen Taktgeber 27 des Gerätes über die Synchronimpulse SP getriggert wird.

Der beschriebene Signalgang ist der Gang für jenes Composite Video Signal, wie es sich das Gerät jeweils selbst erzeugt. Zur Weiterleitung dieses selbsterzeugten Signals dient, wie schon erwähnt, ein zusätzlicher Geräteausgang 30 (Composite Video Output), der zu einem für alle Geräte gemeinsamen Composite Video Bus führt. In der Figur 6 ist dieser zusätzliche Signalpfad durch Abgriffe 51, 52, 53 gekennzeichnet. Die Abgriffe 51 und 52 liegen dabei an den Signalpfaden 41 und 42 vor Schaltern 54 und 55, die dem Decoder 44 für das y-Konfigurationssignal und dem Zeichengenerator 45 vorgeschaltet sind. Der Abgriff 53 am Signalpfad 43 liegt hingegen am Ausgang des Digital-Analog-Umsetzers 47 vor einem Schalter 56. Die Schalter 54 bis 56 verbinden im Normalfall die Signalpfade 41, 42 und 43 unmittelbar mit den systemeigenen Bilderzeugern 44 bis 49. Sie besitzen jedoch auch noch eine zweite Schaltstellung, in der sie jeweils in der dargestellten Weise an die Signalpfade 57, 58 und 59 des Composite Video Input 31 umschaltbar sind. Bei Umschaltung auf den Input 31 wird also die Signalzuführung vom systemeigenen Signalerzeuger 23, 24 zu den Bauelementen 44 bis 49 unterbrochen. An Stelle des eigenen Composite Video Signals wird dem Oszilloskop jetzt das Composite Video Signal eines fremden (insbesondere alarmgebenden) Gerätes aufgeschaltet. Das systemeigene Componente Video Signal wird jedoch gleichzeitig davon ungehindert weiter dem composite Video Output 30 zugeleitet.

Eine typische Anwendung der Erfindung auf eine Kettenschaltung an einzelnen Überwachungsgeräten ohne eigene Zentrale zeigt die Figur 7. Hier sind beispielsweise insgesamt vier Bedside-Geräte 60 bis 63 dargestellt, von denen jedes so aufgebaut ist und ein solches Composite Video Signal erzeugt, wie es in den Erläuterungen zu den vorhergegangenen Figuren 5 und 6 geschildert wurde. Alle vier Geräte dieser Gerätekette (die Zahl der Geräte kann noch beliebig erhöht werden) sind untereinander über insgesamt drei Leitungen 64, 65, 66 verbunden (bzw. über Schalter zusammenschaltbar, wie etwas weiter unten noch näher erläutert wird). So ist z. B. die Leitung 64 der gemeinsame Signal Bus (CVB) für das Composite Video Signal (CV). Die Leitung 65 ist der gemeinsame Alarm Bus und die Leitung 66 ist schließlich die gemeinsame Übertragungsleitung für den Synchronimpuls SP zur Synchronisierung der x-Ablenkung aller Geräte 60 bis 63. Jedes der Geräte trägt an der Frontseite (schematisch angedeutet) je eine LED-Taste 67 bis 70.

In jeder dieser Tasten ist also eine Leuchtdiode (LED) integriert. Jede dieser Leuchtdioden steht wiederum schaltungsmäßig mit dem Alarm Bus 65 in solcher Verbindung, daß sie innerhalb der Taste aufleuchtet, wenn von einem anderen Gerät der Kette ein Alarmsignal erzeugt und auf den Alarm Bus ausgegeben wird. Im alarmgebenden Gerät selbst wird allerdings die dortige LED nicht aktiviert. Hier wird die Alarmsituation optisch und akustisch mit gesonderten Anzeigemitteln angezeigt. Der Arzt oder die überwachende Schwester können dann sofort unterscheiden, ob das betreffende Gerät selbst Alarm gibt, d. h. ob der daran angeschlossene Patient selbst der Alarmpatient ist oder ob ein anderes Gerät der Kette mit dem dort angeschlossenen Patienten Gegenstand des Alarmes ist.

Was die Zuleitung von Composite Video Signalen von den Geräten 60 bis 63 zum Composite Video Bus 65 bzw. die Entnahme solcher Signale durch einzelne Geräte aus dem Composite Video Bus 65 betrifft, so sind in der Figur 7 innerhalb der Blocks, die die Geräte 60 bis 63 symbolisieren, wiederum nur rein symbolisch Schalter 71 bis 78 eingezeichnet. Die Schalter 71 bis 74 (CVO-Schalter) sind in den Leitungen 30 (siehe Figuren 5 und 6) der Video Signal Outputs (CVO) der Geräte angeordnet. Die Schalter 75 bis 78 (CVI-Schalter) liegen in den Leitungen 31 (siehe Figuren 5 und 6) der Video Signal Inputs (CVI). Ein geschlossener CVO-Schalter 71 bis 74 bedeutet also, daß das betreffende Gerät 60, 61, 62 oder 63 speziell sein Composite Video Signal, das es in diesem Moment auch gleichzeitig am Bildschirm des eigenen Oszilloskops 79, 80, 81 oder 82 abbildet, in den Composite Video Bus 64 einspeist. Ein geschlossener CVI-Schalter 75 bis 78 bedeutet, daß jenes Composite Video Signal, das gerade von einem anderen Gerät in den Composite Video Bus 65 eingespeist wird, vom betreffenden Gerät über dessen Video Signal Input aus dem CV-Bus übernommen und am Bildschirm des Oszilloskops 79, 80, 81 oder 82 dieses Gerätes anstelle der Abbildung des eigenen Composite Video Signals abgebildet wird.

Im Ausführungsbeispiel der Figur 7 gibt z. B. das Gerät 63 Alarm. Dieser Alarm teilt sich über den Alarm Bus 65 den Geräten 60, 61 und 62 (oder allen weiteren Geräten, falls solche zusätzlich noch in der Kette angeschlossen sind) mit. Die LED-Tasten 67, 68 und 70 dieser Geräte leuchten also, was in der Figur 7 durch symbolische Lichtstrahlen am Rand der Tasten angedeutet ist. Gemäß der vorhergehenden Ausführungen ist außerdem der CVO-Schalter 74 des Gerätes 63 im Augenblick der Alarmgebung geschlossen worden. Die CVO-Schalter 71, 72 und 73 der Geräte 60, 61 und 63 bleiben hingegen geöffnet, da in diesen Geräten ja kein Alarm erzeugt wurde. Demnach wird also in den Composite Video Bus 64 das Composite Video Signal des alarmgebenden Gerätes 63 eingespeist. Dieses Composite Video Signal kann nun von einem beliebigen Gerät 60 bis 63 durch Drücken der LED-Taste 67 bis 69 aus dem CVB 64 entnommen werden. Das Drücken einer leuchtenden LED-Taste führt dazu, daß das Dauerleuchten in ein Blinken übergeht. Damit wird angezeigt, daß auf dem Bildschirm des Oszilloskops des betreffenden Gerätes nicht mehr das eigene, sondern ein fremdes Signalbild abgebildet wird. Das Blinken einer Taste wird in Figur 7 symbolisch durch Strahlen angedeutet, die radial in das Tastenzentrum laufen.

Im Ausführungsbeispiel der Figur 7 wurde z. B. die LED-Taste 67 des Gerätes 60 gedrückt. Der CVI-Schalter 75 dieses Gerätes wurde demnach geschlossen und das CV des alarmgebenden Gerätes 63 gelangt somit über den Composite Video Input des Gerätes 60 in den CV-verarbeitenden Signalteil dieses Gerätes und von diesem entsprechend zum Bildschirm des Oszilloskops 79.

Die beschriebenen Schaltabläufe der Schalter 71 bis 78 sind von rein exemplarischem Charakter. Es gibt hier beliebige Modifikationen, die eingesetzt werden können, ohne daß der prinzipielle Ablauf der CV-Ausgabe durch ein Gerät in den CV-Bus bzw. der Aufnahme von CV-Signalen aus dem CV-Bus durch ein anderes oder mehrere andere Geräte maßgeblich geändert wird. So ist es also durchaus möglich, daß abweichend von vorstehend beschriebener Weise z. B. über ständig geschlossene CVO-Schalter ständig alle CV-Signale aller Geräte in den CV-Bus eingespeist werden. Erst mit Auftreten eines Alarmsignals im gemeinsamen Alarm Bus werden sämtliche CVO-Schalter geöffnet, bis auf jenen des alarmgebenden Gerätes. Es wird dann also im Alarmfall, wie erwünscht, in den CV-Bus nur noch das alarmbehaftete CV eingespeist.

Im Vergleich mit dem Prinzipschaltbild der Figur 6 kann der jeweilige COI-Schalter 75, 76, 77 oder 78 die drei Umschalter 54, 55, 56 symbolisieren. Ein offener COI-Schalter der Figur 7 entspräche also der ersten Schaltposition der Schalter 54, 55, 56 in Figur 6, in der die geräteeigenen CV-Erzeuger 23, 24 etc. über die Leitungen 41, 42, 43 direkt mit den bildaufbauenden Bausteinen 44 bis 49 verbunden sind. Ein geschlossener COI-Schalter der Figur 7 gäbe hingegen die zweite Schaltposition wieder, in der diese direkten Verbindungen unterbrochen und dafür die bildaufbauenden Bausteine (bis auf den A/D-Umsetzer 47) mit den Signalleitungen 57, 58, 59 des CVI verbunden sind. Der jeweilige COI-Schalter kann jedoch z. B. auch als zusätzlicher Steuerschalter für die Schalter 54, 55, 56 ausgebildet sein, der die Schalter 54, 55, 56 umschaltet, sobald er mit dem Drücken der LED-Taste geschlossen wird. In besonderer Ausbildung und unabhängig von vorstehend beschriebenen Funktionsmöglichkeiten kann der COI-Schalter auch integrales Schaltkontaktteil der LED-Taste sein.

Was die Priorität von Alarmen betrifft, so sind die Schalter auch wie folgt gesteuert :

Sofern von einem einzelnen Gerät ein Alarm gegeben wird, so ergibt sich der vorstehend beschriebene Funktionsablauf. Wird hingegen von zwei oder noch mehr Geräten Alarm gegeben, so tritt der vorstehend beschriebene Funktionsablauf grundsätzlich nur für jenes Gerät ein, das von allen alarmgebenden Geräten zuerst Alarm erzeugt hat. Nur für diese Gerät schließt sich also der betreffende CVO-Schalter ; die CVO-Schalter der anderen alarmgebenden Geräte bleiben hingegen geöffnet, solange bis der Alarm des ersten Gerätes zurückgesetzt wurde, z. B., durch Drücken einer Alarm-Rücksetztaste. Sobald dies geschehen ist, tritt an die Stelle des Gerätes, das zuerst Alarm gegeben hat, ein nächstfolgendes Gerät, das Alarm gibt (bei mehreren gleichzeitig alarmgebenden Geräten z. B. das Gerät mit der niedrigsten Bettnummer, dann das mit der nächsthöheren Bettnummer etc.).

Signifikante Eigenschaft einer Kettenschaltung, wie sie im Prinzip in der Figur 7 dargestellt ist, ist also, daß jedes Gerät innerhalb der Kette die Funktion eines Zentralgerätes übernehmen kann, das in der Zentralfunktion dann also von einem beliebigen anderen Gerät, das z. B. gerade Alarm erzeugt, dessen Composite Video Signal zur Aufzeichnung auf dem Bildschirm des eigenen Oszilloskops abrufen kann. Wesentlich ist nur, daß zur Übernahme der Zentralfunktion am entsprechenden Gerät eine LED-Taste gedrückt wird.

Nach einem etwas modifizierten Prinzip funktioniert hingegen eine Anordnung aus Bedside-Geräten, für die von vornherein zur Steuerung des Gesamtablaufes eine Zentraleinheit vorgesehen ist. Eine solche Anordnung ist in der Figur 8 im Prinzipschaltbild dargestellt. In dieser Anordnung sind einer Zentrale 83 insgesamt n Einzelgeräte (Bedside-Units) zugeordnet, die mit der Zentrale sternförmig über geräteseitige Composite Video Busse für geräteseitige Composite Video Signale CVB 1 bis CVB n und Alarm Busse für geräteseitige Alarme AL 1 bis AL n verbunden sind. Ein einzelner zentraler Composite Video Bus (Composite Video Central) für ein zentrales Composite Video Signal CVC führt von der Zentrale 83 zu den Bedside-Geräten zurück. In kleinster Einheit sind in Sternschaltung einer Zentrale 83 vorzugsweise n = 4 Einzelgeräte zugeordnet. Die vorliegende Schaltung der Figur 8 beinhaltet jedoch z. B. n = 16 Einzelgeräte in Sternschaltung mit der Zentrale 83, von denen jedoch lediglich die beiden ersten Bedside-Geräte dargestellt sind. Diese beiden Geräte sind mit den Kennziffern 84 und 85 belegt. Die n = 16 Einzelgeräte können bei Bedarf in Untergruppen von vorzugsweise 4 × 4 Bedside-Geräten unterteilt sein. Jeder Gruppe ist dann z. B. ein eigener Wandanschluß (Wall-Terminal) in Richtung Zentrale zugeordnet. Jedes der Bedside-Geräte der Sternformation mit Zentrale ist im Prinzip genauso aufgebaut, und es funktioniert hinsichtlich der Erzeugung seines Signals CVB 1 bis CVB n genauso, wie es für die Figuren 5 und 6 beschrieben wurde. Die Blocks 86 und 87 innerhalb der Geräteblocks 84 und 85 symbolisieren also im wesentlichen die Zusammenfassungen jener Bauelemente 41 bis 56 des Bildverarbeitungs- und Bildaufzeichnungsteils der Geräte, wie sie insbesondere in Figur 6 detailliert sind. Die Blocks 88 und 89 deuten hingegen den Bildwiederholungsspeicher 24 mit zugehörigem Schieberegister 23, Adressrechner 25, Taktgeber 27 etc. an, so wie diese Bauelemente im Prinzip in der Figur 6 ebenfalls erläutert sind.

Auch was die Alarmgebung und Alarmbenachrichtigung betrifft, laufen vom Prinzip her dieselben Mechanismen ab, wie sie für die Kettenschaltung der Figur 7 beschrieben wurden. Der einzige, hier jedoch sehr wesentliche Unterschied besteht darin, daß allen Einzelgeräten 84, 85 etc. bereits eine Zentrale 83 zugeordnet ist, die von vornherein und allein die Zentralfunktion übernimmt. Eine Übernahme der Zentralfunktion durch ein beliebiges Bedside-Gerät, wie es bei der Schaltung von Bedside-Geräten in Kettenformation (Figur 7) möglich ist, ist bei der Sternschaltung also nicht möglich. Daraus resultiert, daß also nicht nur sämtliche Composite Video Signale CVB 1 bis CVB n der einzelnen Bedside-Geräte über einzelne Composite Video Busse 90, 91 (für die beiden ersten Busse), 92 (für den 16-ten Bus) sternförmig zur Zentrale 83 geführt werden ; dasselbe geschieht auch mit Alarmen, da jedes einzelne Bedside-Gerät 84, 85 der Sternformation ebenfalls in Sternform direkt über einen geräteeigenen Alarmbus mit der Zentrale 83 verbunden ist. Von den insgesamt sechzehn Alarmbussen sind die beiden ersten Busse mit 93, 94 und der 16-te Bus mit 95 angedeutet. Wird von einem der sechzehn Bedside-Geräte 84, 85 etc. ein Alarm erzeugt, so wird dieser Alarm der Zentrale 83 zugeführt.

Die Zentrale 83 selbst ist, was dem zentralen Bildverarbeitungs- und Bildaufbauteil 96 betrifft, im Prinzip genauso aufgebaut wie ein Bedside-Gerät. Sie umfaßt entsprechend einem Bedside-Gerät auch einen Wiederholungsspeicher 97 und einen zentralen Mikroprozessor 98. Anstelle von Einschubmodulen mit entsprechendem Datenverkehr sind jedoch in der Zentrale 83 auf passenden Ergänzungskarten ein zentraler Signal- und Daten-Multiplexer 99, ein Alarmmultiplexer 100 und eine Steuereinrichtung 101 für Daten- und Adress-Steuerung zwischen zentralem Multiplexer 99 und zentralem Bildwiederholungsspeicher 97 eingefügt. Der zentrale Signal- und Daten-Multiplexer 99 besitzt die Eigangsleitungen 90, 91 etc. bis 92 für die insgesamt sechzehn Composite Video Signale CVB 1 bis CVB 16 der sechzehn einzelnen Bedside-Geräte. Er ist ausgangsseitig über Leitungen 102 bis 106 für Signale A, B, C, D, CH mit der Steuereinrichtung 101 verbunden, die wiederum ausgangsseitig über Datenbusse 107 und 108 sowie über Adressenbusse 109 und 110 mit dem zentralen Bildwiederholungsspeicher 97 und dem zentralen Mikroprozessor 98 in Verbindung steht. Darüber hinaus führt eine weitere Ausgangsleitung 111 des zentralen Multiplexers 99 über einen Schalter 112 direkt zum Signaleingang des Bildverarbeitungs- und Bildaufzeichnungsteils 96 der Zentrale. Über diese Schaltleitung empfängt das Bildteil 96 der Zentrale das komplette Composite Video Signal CVB eines beliebig anwählbaren Bedside-Gerätes der Sternkonfiguration. Schließlich besitzt der zentrale Multiplexer 99 auch noch drei Ausgänge 113, 114 und 115 für Recordersignale REC 1, REC 2, REC 3, die bei Bedarf auf zwei in der Zentrale integrierten Signalrecordern 116, 117 sowie einem extern anzuschließenden Signalrecorder (Anschließpfeil 118) registrierbar sind. Zur Synchronisierung der Recorder dienen Synchronisiersignale Synch 1, Synch 2, Synch 3, die an die Ausgangsleitungen 119, 120 und 121 des zentralen Multiplexers 99 anfallen. Die Signale REC 1, REC 2, REC 3 werden zusammen mit den Synchronisiersignalen Synch 1, Synch 2, Synch 3 in Decodierern 122, 123, 124 decodiert und dann mittels Schalter 125, 126, 127 den Recordern aufgeschaltet.

Der Alarmmultiplexer 100 besitzt einen Ausgangsalarmbus 128 zum zentralen Prozessor 98. Der zentrale Prozessor 98 steht über eine Daten- und Adress-Leitung 129 mit dem Bildteil 96 der Zentrale in Verbindung, und er steht außerdem über Leitungen 130, 131 in ständiger Kommunikation mit der Steuereinrichtung 101.

Ein weiterer, sehr wesentlicher Bestandteil ist der zentrale Composite Video Bus 132. Über diesen zentralen Bus wird das Composite Video Signal CVC der Zentrale 83 zu den Bedside-Geräten geführt (bzw. auch in Richtung auf die Recorder 116, 117 etc. gegeben). Bei Bedarf (wieder durch Drücken einer LED-Taste wie bei Kettenschaltung gemäß Figur 7) kann das zentrale Composite Video Signal jederzeit aus dem zentralen Bus 132 in ein Bedside-Gerät abgerufen werden. Hierzu muß lediglich wieder ein Schalter 133, 134 etc. (LED-Taste) umgelegt werden. Das Composite Video Signal CVC gelangt dann über den Composite Video Eingang des betreffenden Gerätes in den Bildverarbeitungsteil. Das eigene Signalbild wird dann durch das Zentralbild ersetzt.

Signifikante Eigenschaft der Sternkonfiguration ist also, daß die Composite Video Signale CVB 1 bis CVB n der Bedside-Geräte 84, 85 etc. sternförmig zur Zentrale 83 geführt werden. Im Normalfall umfaßt die Zentrale 83, jedoch ebenfalls nur ein einziges Oszilloskop mit maximal vier Kanälen. Damit lassen sich am Bildschirm des zentralen Oszilloskops also höchstens die vier Kanäle eines einzigen Darstellungsgerätes 86 oder 87 etc. als ausgewähltes Signal CVB über die Leitung 111 übernehmen. Ein zeitgestaffeltes Umschalten des zentralen Anzeigegerätes 96 auf andere Darstellungsgeräte ist durch den Zeitmultiplex des Multiplexers 99 zwar möglich und wird so auch durchgeführt ; Schwierigkeiten bereitet jedoch die Durchführung eines Abbildungsverfahrens, bei dem in gemischter Form Signale bestimmter Kanäle von Bildgeräten 86, 87 etc. unterschiedlicher Bedside-Geräte 84, 85 auf dem zentralen Bildgerät 96 dargestellt werden sollen. In unterschiedlichen Bedside-Geräten können die verschiedenen Bildgeräte mit sehr unterschiedlichen Ablenkgeschwindigkeiten der x-Ablenkung arbeiten. So gibt es z. B. Geräte, die mittels Geschwindigkeits-Wahlschalter auf eine höchste Ablenkgeschwindigkeit von z. B. 50 mm/s, und andere, die auf eine geringste Ablenkgeschwindigkeit von z. B. 12,5 mm/s geschaltet sind. Jedes Gerät kann zumindest zwischen diesen beiden Ablenkgeschwindigkeiten umgeschaltet werden. Die gleichzeitige Abbildung von Signalspuren mit unterschiedlicher Ablenkgeschwindigkeit ist jedoch praktisch kaum durchführbar.

Die Sternschaltung der Figur 8 bewältigt jedoch auch diese Situation und zwar durch einen besonderen Trick. Dieser Trick ist durch folgende Aspekte gekennzeichnet :

Der erste Aspekt berücksichtigt den Umstand, daß an jedem Bildwiederholungsspeicher im Bildverarbeitungsteil eines einzelnen Bedside-Gerätes mit jeder Eingabe eines neuen Meßwertes ein ältester Meßwert anfällt, der aus der Signaldarstellung eliminiert wird. Der Zeitpunkt des Rauswurfes dieses jeweils ältesten Meßwertes wird nun mit der Periode einer Signaldarstellung so synchronisiert, daß er in die Rückführphase (retrace time) der x-Ablenkung fällt (zweiter Aspekt). Der älteste Meßwert wird damit also nicht mehr im Signalbild abgebildet ; er steht jedoch als verzögerter Meßwert (delayed data) zur Abbildung auf einem anderen Bildgerät zur Verfügung, falls eine solche verzögerte Abbildung gewünscht wird. Diesen Umstand macht sich nun die Zentrale 83 in der Sternschaltung der Figur 8 zunutze (dritter Aspekt). Diese Zentrale umfaßt einen Bildwiederholungsspeicher 97, der über den Multiplexer 99 von allen Geräten im Zeitmultiplex verzögerte Meßwerte A, B, C und D erhält. Die verzögerten Meßwerte werden von der Steuereinrichtung 101 zusammen mit den Charakteradressen CA im Bildwiederholungsspeicher eingeschrieben. Von dort können sie zu beliebiger Zeit und in beliebiger Mischung der Signalspuren wieder abgerufen und auf dem zentralen Darstellungsgerät 96 als Mischbild dargestellt werden.

Der beschriebene Trick macht es also möglich, daß an einem zentralen Oszilloskop innerhalb der Zentrale 83, das z. B. vier Kanäle aufweist, in beliebig wechselnder Reihenfolge insgesamt vier unterschiedliche Kanäle aus Oszilloskopen von vier unterschiedlichen Bedside-Geräten dargestellt werden können.

Tritt nun an einem der externen Geräte ein Alarm auf, so gelangt dieser Alarm über den entsprechenden Alarm Bus AL 1 bis AL n zum Alarmmultiplexer 100 und von dort zum zentralen Prozessor 98. Der zentrale Prozessor 98 steuert nun den Signaltransfer zwischen Bildwiederholungsspeicher 97 und Darstellungsgerät 96 in der Weise, daß die verzögerten Werte der alarmgebenden Signalspur (z. B. alarmgebendes EKG) anstelle einer vorherigen Signalwertaufzeichnung in den Kanal mit der höchsten Nummer, d, h. also in den vierten Kanal, eingeschrieben wird. Tritt eine zweite alarmgebende Signalspur auf, so wird diese entsprechend in den nächstniedrigeren Kanal, d. h. also in den dritten Kanal, etc eingeschrieben. Gleichzeitig mit der Darstellung der alarmgebenden Signalspur wird vom Bildwiederholungsspeicher die Nummer des Bettes überspielt, an dem das alarmgebende Gerät steht. Die Bettnummer wird am zentralen Bildschirm durch Blinken hervorgehoben, so daß die überwachende Person an der Zentrale sofort erkennt, daß ein Alarmfall an einem Bedside-Gerät mit bestimmter Bettnummer vorliegt.

Der Alarmfall kann von der Zentrale 83 über den zentralen Bus 132 auch anderen nicht alarmgebenden Bedside-Geräten mitgeteilt werden. Hier leuchtet dann z. B. wieder entsprechend vorhergehender Beschreibung eine LED-Taste auf. Durch Drücken einer solchen Taste kann dann das zentrale Composite Video Signal CVC auf den Bildschirm des Oszilloskops des betreffenden Gerätes unter Abschaltung der eigenen Bilddarstellung übernommen werden.

Die Figur 9 zeigt in detaillierter Darstellung den inneren prinzipiellen Aufbau des Multiplexers 99, so wie er nach Aufbau und Funktionsweise im Zusammenhang mit der Sternschaltung der Figur 8 vorher schon erläutert wurde. Die Verteilung der verschiedenen Signale im Multiplexbetrieb auf die unterschiedlichen Ausgangsleitungen übernehmen gebräuchliche Analogschalter 135 bis 146 im Innern des Multiplexers.

Die Figur 10 zeigt den inneren Aufbau der Steuereinrichtung 101 gemäß Figur 8 in der Verschaltung zwischen Multiplexer 99 einerseits und Bildwiederholungsspeicher 97 und zentralem Prozessor 98 andererseits. Die Steuereinrichtung umfaßt, was die orts- und zeitgerechte Weiterleitung der verzögerten Daten A bis D betrifft, vier Einzelblöcke 147 bis 150, von denen jeder im Prinzip im Innern so aufgebaut ist, wie es für den Block 147 im Detail dargestellt ist. Jeder der Blöcke 147 bis 150 umfaßt also ein Schieberegister 151 für Datenwörter (data word shift register), einen Zähler 152 (strobe counter) zum Takten des Schieberegisters, der einen Clock-Eingang 153 beinhaltet, einen Synchronisierer 154 (synchronizer) für den Zähltakt und einen Wort-Zähler 155 (word counter) für die Adressen. Die Ausgangsdaten des Daten-Schieberegisters 151 gehen über einen Ausgang 156 auf die Datenbusse 107, 108 für Bildwiederholungsspeicher 97 und zentralen Prozessor 98. Die Ausgangsadressen des Wort-Zählers 155 gehen entsprechend über einen Ausgang 157 zu den Adress-Bussen 109, 110 für Bildwiederholungsspeicher und zentralen Prozessor.

Die Steuereinrichtung 101 umfaßt darüber hinaus für die Weiterleitung von Zeichenadressen CH wieder ein Schieberegister 158 für Datenwörter mit Ausgang 159 zu den Daten-Bussen 107, 108 und einen Wort-Zähler 160 mit Ausgang 161 zu den Adress-Bussen 109, 110. Der Block 162 umfaßt wieder eine Synchronisier- und Steuereinrichtung (four cycle synchronization and control) mit Starteingang 163 zum Starten des Lesens eines Zeichens (start character read) und Eingang 164 für den Zeittakt, der den Bildaufbau des Zeichens kontrolliert (timing from display control, siehe hierzu nochmals die Figur 6 in Verbindung mit Figur 5). Ein Ausgang 165 führt zu einer Kontrolleinheit 166 (DMA Control Arbitrator and Multiplexer Control), die über die Leitungen 130 und 131 in Kommunikation mit dem zentralen Prozessor steht.

Wie schon erwähnt, werden zum Mischen von Signalspuren verzögerte Daten (delayed data) verwendet, die in den jeweiligen Rückführphasen (retrace time) der x-Ablenkung anfallen. Die Zeitdauer dieser Rückführphase beträgt 960 µs, was in der Figur 11 oben zur Erinnerung an Figur 4 nochmals belegt wird. Abweichend von der Figur 4 ist in der Figur 11 unterhalb des schon beschriebenen Zeitdiagrammes jedoch noch ein Impulsplan eingezeichnet, der rein schematisch die Datenübertragung im Feld IV während der Rückführphase der x-Ablenkung abhandeln soll. Die Datenübertragung erfolgt in digitaler Form. Wie schon beim y-Konfigurationswort des Phase I' wird auch bei den digitalen Signalen der Retrace time IV der Zustand NULL (« 0 ») durch das Auftreten des Spannungsniveaus + 0,75 V und der Zustand EINS (« 1 ») durch das Auftreten des Spannungsniveaus — 1V im Composite Video Signal belegt. In der Rückführphase IV können nun bis zu zehn Digitalworte übertragen werden, von denen jedes 8 bit aufweist. In der Rückführphase IV wird bis auf die Zeiten der Übertragung digitaler Worte eine NULL (« 0 ») übermittelt, d. h. das Composite Video Signal befindet sich auf dem Niveau + 0,75 V. Jeweils etwa 50 µs nach Beginn der Rückführphase wird mit der Übermittlung von digitalen Worten begonnen. Jedes zu übermittelnde Wort wird mit einem Startbit 167 eröffnet, das 2 µs lang ist und das eine EINS (« 1 ») signalisiert. Auf dieses Startbit folgt dann jeweils ein Wort 168, das aus 8 bit's 169 bis 176 besteht, von denen jedes ebenfalls 2 µs lang ist. Die einzelnen bit's 169 bis 176 können NULL oder EINS-Status aufweisen, je nach Gehalt des Wortes. In der Figur 11 sind diese Wechselmöglichkeiten zwischen NULL und EINS schematisch durch je zwei Diagonalen in jedem bit angedeutet. In jedem Wort ist das jeweils erste bit mit LSB und das jeweils letzte bit mit MSB bezeichnet. LSB steht für « Least Significant Bit » und MSB für « Most Significant Bit ». Damit ist für jedes Wort die Richtung festgelegt, in der die bit's dieses Wortes zu lesen sind. Innerhalb der Rückführphase IV von insgesamt 960 µs werden also bis zu zehn derartiger 8-bit-Worte übermittelt, wobei jedes neue Wort durch ein neues Startbit angekündigt und eingeleitet wird. Zwischen Ende des letzten Wortes einer solchen Folge und Ende der Rückführphase liegen darüber hinaus noch ca. 60 µs an Reservezeit.

In der Figur 11 ist eine solche Folge von z. B. zehn Worten zu je 8 bit mit der Kennziffer 177 angedeutet. Das jeweils erste Wort einer solchen Folge, das immer übermittelt wird, ist ein Index Wort. Dieses Index Wort, das in Figur 11 im Detail in der untersten Darstellung unter der Kennziffer 178 in seine 8 bit's aufgegliedert dargestellt ist, gibt Information über Ablaufgrößen für die Aufzeichnung, wie z. B. über Aufzeichnungsgeschwindigkeit (speed), die Kanalauswahl (channel CHAN 1 to CHAN 4 bzw. CHAN 1' to CHAN 4'), das Vorhanden- oder Nichtvorhandensein von Trendwerten (trend points), das Vorliegen von aktuellen und/oder verzögerten Meßwerten (real time data, delayed data) etc. Die auf das Index Wort folgenden weiteren ersten vier Worte definieren verzögerte Signalwerte für die vier Kanäle CHAN 1, CHAN 2, CHAN 3 und CHAN 4 und sind in dieser Bezeichnung in der Wortgruppe 177 auch eingetragen. Bei niedrigster Signalübertragungsgeschwindigkeit fällt pro Rückführphase normalerweise nur je ein ältester Meßwert an. Bei höchster Geschwindigkeit können jedoch bis zu zwei älteste Meßwerte anfallen. Diese zuletzt genannte Möglichkeit berücksichtigt eine Wortgruppe 177 durch vier weitere Worte, die in der Figur 11 mit CHAN 1', CHAN 2', CHAN 3', CHAN 4' gekennzeichnet sind. Sofern als pro Rückführphase insgesamt zwei verzögerte Meßwerte anfallen, so findet sich das jeweilige Paar in den Wortpaaren CHAN 1 und CHAN 1', CHAN 2 und CHAN 2' etc. Das zehnte und letzte Wort der Wortgruppe 177 ist schließlich ein Wort für einen Trendwert, sofern ein solcher Trendwert vorher von einem Trendverwerter, insbesondere Trendrecorder, über das Indexwort angefordert worden war.

Zusammen ergibt sich also das folgende Schema einer Wortgruppe mit z. B. zehn Worten :

| | | |
|---|---|---|
| Wort 1 : | Index Wort | wird immer übermittelt |
| Wort 2 : | CHAN 1 | belegt bei niedrigster Anfallrate 1 Wort/9,778 ms (12,5 mm/s) und |
| Wort 3 : | CHAN 2 | bei höchster Anfallrate 2 Worte/4,884 ms (50 mm/s) |
| Wort 4 : | CHAN 3 | |
| Wort 5 : | CHAN 4 | |
| Wort 6 : | CHAN 1' | nicht belegt außer bei höchster Anfallrate von 2 Worten/4,884 ms |
| Wort 7 : | CHAN 2' | (50 mm/s) |
| Wort 8 : | CHAN 3' | |
| Wort 9 : | CHAN 4' | |
| Wort 10 : | Trendpunkt | nur übermittelt bei Anforderung durch Trendrecorder |

Das 8 bit-Indexwort setzt sich beispielsweise wie folgt zusammen :

BIT's 7 und 6

Sie legen die Rate für neueste Meßwerte nach folgender Tabelle fest.

| BIT 7 | BIT 6 | |
|---|---|---|
| 0 | 0 | es liegt kein Meßwert vor |
| 0 | 1 | 1 Meßwert pro Kanal und pro 9,778 ms |
| 1 | 0 | 1 Meßwert pro Kanal und pro 4,884 ms |
| 1 | 1 | 2 Meßwerte pro Kanal und pro 4,884 ms |

BIT's 5 und 4

Sie legen die Kanalnummer nach folgender Tabelle fest :

| BIT 5 | BIT 4 | |
|---|---|---|
| 0 | 0 | Oberer Kanal CHAN 1 |
| 0 | 1 | Mittlerer Oberer Kanal CHAN 2 |
| 1 | 0 | Mittlerer Unterer Kanal CHAN 3 |
| 1 | 1 | Unterer Kanal CHAN 4 |

BIT 3

Dieses BIT legt fest, ob ein Trendwort für den nächsten Kanal vorhanden ist.

BIT 3 = 0 kein Trendwort vorhanden
BIT 3 = 1 Trendwort vorhanden

BIT's 2 und 1

Sie legen fest, ob die Daten verzögerte Daten (delayed data) oder Echtzeitdaten (real time data) sind.

(Siehe Tabelle, Seite 13 f.)

| BIT 2 | BIT 1 | |
|---|---|---|
| 0 | 0 | normal, alle Daten sind verzögerte Daten (delayed data) |
| 0 | 1 | Kanal 4 gestoppt, Aufzeichnung von Echtzeitdaten (Transfer mode) |
| 1 | 0 | Kanal 4 in Cascade Mode |
| 1 | 1 | alle Kanäle gestoppt, Aufzeichnung von Echtzeitdaten (real time data) |
| BIT 0 | | |
| 1 | | Übertragung ist gültig |
| 0 | | Übertragung ist ungültig |

Wie schon erwähnt, wird ein Trendwort nur dann übermittelt, wenn die Übermittlung angefordert wurde. Diese Anforderung kann von einem Recorder kommen, der dem Bedside-Gerät beigeordnet ist. Sie kann aber auch via UART durch einen zentralen Recorder der Zentralstation kommen (siehe hierzu nochmals Beschreibung zu Figur 5).

Die Figur 12 zeigt den mechanischen Prinzipaufbau eines elektromedizinischen Überwachungsgerätes mit vier Einschubmodulen, so wie es in Verbindung mit der Erfindung bevorzugt als Bedside-Gerät eingesetzt wird. Das Gerät umfaßt also ein Gerätegehäuse 179 mit der Frontfläche 180. Auf der rechten Seite der Frontfläche ist eine Öffnung für eine Ausnehmung im Innern des Gerätegehäuses mit 181 angedeutet. Durch diese Öffnung 181 können in die Ausnehmung insgesamt vier Einschubmodule eingeschoben werden, die hier in Übereinstimmung mit dem Geräteprinzipschaltbild der Figur 5 wieder mit 1, 2, 3 und 4 beziffert sind. Das Gerät ist mit einem x, y, z-Oszilloskop ausgerüstet, dessen Bildschirm mit 182 angedeutet ist. Die Elemente 184, 185 an der Frontplatte sind Bedien- und Anzeigeelemente, wie Tastenschalter, LED-Anzeigefelder etc. Ihre Anordnung ist rein schematisch dargestellt. Das Element 186 deutet die früher schon beschriebene LED-Leuchttaste für die Anzeige und Übernahme des fremden Alarmes an. Das Element 187 kennzeichnet z. B. die Rücksetztaste für einen Alarm im eigenen Gerät.

Das in der Figur 12 dargestellte Gerät ist wieder speziell ein elektromedizinisches Gerät. Die Einschubmodule 1 bis 4 sind also Teil des Signalübertragungssystems für physiologische Signale, die mittels geeigneter Abnehmer am Körper eines Patienten abgenommen werden. Zu diesem Zwecke werden also (nicht dargestellte) Abnehmer am Körper des Patienten positioniert und über ein Signalkabel (ebenfalls nicht dargestellt) am jeweiligen Einschub angekoppelt. Die Einschübe umfassen zu diesem Zweck also Ansteckbuchsen 188 bis 191 für entsprechende Stecker der Signalkabel. Die restlichen Elemente 192 bis 195 sind in wieder nur rein schematischer Andeutung Datentasten oder LED-Anzeigefelder der Einschubmodule. Im Beispiel der Figur 12 ist beispielsweise der unterste Enschub 4 ein $CO_2$-Einschub, die beiden mittleren Einschübe sind z. B. Einschübe für Blutdruck- und Temperaturmessung und der obere Einschub 1 dient z. B. zur EKG-Messung. Das abgebildete Gerät kan zusammen mit weiteren gleichartigen Geräten zu einer Kette mit Aufbau und Funktionsweise, wie sie in der Figur 7 dargestellt ist, zusammengeschaltet werden. Ebensogut kann das beschriebene Gerät mit einer entsprechenden Zahl weiterer Geräte zusammen mit einer Zentrale zu einer Sternschaltung zusammengefügt werden, wie sie in der Figür 8 dargestellt ist.

Die Reihenfolge, in der im Gerät der Figur 12 die einzelnen Einschubmodule 1 bis 4 angeordnet sind, ist völlig beliebig.

Dies wird in einfachster Weise gemäß den Figuren 13 bis 15 dadurch gewährleistet, däß dem vorverarbeitenden Mikroprozessor im Gerät auf der Seite der Einschubmodule 1 bis 4 in Signalübertragungsrichtung vor jeder Koppelstelle 8, 9 wenigstens je ein Analog-Digital-Konverter 196 pro Einschub 1 bis 4 zugeordnet ist. Völlig unabhängig davon, von welcher Art die Einschubmodule sind und in welcher Reihenfolge bzw. räumlicher Zuordnung zueinander die Einschubmodule in der Einbuchtung des Gerätes eingesteckt sind, werden nun von der Seite des Gerätes her die einzelnen Analog-Digital-Konverter 196 sämtlicher eingesteckter Module 1 bis 4 nach einem fest vorgegebenen Rhythmus zur Konversion getaktet. Die Zuordnung einzelner konvertierter Signalwerte zu einem Modul erfolgt auf der Seite des Verarbeitungsteiles in einfachster Weise durch ein Identifikationssignal für jeden einzelnen Modul 1 bis 4, das zeitverschachtelt zusammen mit den konvertierten Signalwerten übermittelt wird.

Im Gesamtschema dieser Übermittlungsweise soll nun der Analog-Digital-Konverter 196 eines jeden Einschubmodules 1 bis 4 möglichst preiswert sein und dazu auch noch gleichzeitig mit möglichst hoher Effektivität arbeiten. Gemäß dem vorliegenden Ausführungsbeispiel der Figuren 13 bis 15 wird dies dadurch gewährleistet, daß Analog-Digital-Konverter 196 eingesetzt werden, die bei einer relativ niedrig vorgegebenen Konversionsrate pro Zeiteinheit hinsichtlich der unterschiedlichen Frequenzen einzelner Signale in den unterschiedlichen Frequenzkanälen mit entsprechend unterschiedlicher Häufigkeit abgetastet werden. Die Figur 13 zeigt beispielsweise im Prinzipschaltbild den wesentlichen Innenaufbau eines IKG-Einschubes mit drei Kanälen 1H, 1A, 1B. Der Kanal 1H ist dabei ein Höchstgeschwindigkeitskanal ; dieser Kanal, der sich nur im EKG-Einschub befindet, wird 2 400 mal in der Sekunde abgetastet. Durch diese hohe Abtastfrequenz werden im EKG solche Vorgänge, die überdurchschnittlich hochfrequent sind z. B. arhythmische Ereignisse oder Pacemaker-Impulse, erfaßt und angezeigt. Der Höchstgeschwindigkeitskanal 1H arbeitet also nur in Verbindung mit einem zusätzlichen Arhythmie-Verarbeitungsteil (z. B. Arhythmie-Computer). Der Kanal 1A ist hingegen der Hauptdatenkanal für das EKG. Dieser Kanal 1A wird insgesamt 400 mal in der Sekunde abgetastet. Der Kanal 1B kann als zweiter Datenkanal für eine weitere Meßgröße dienen. Dieser Kanal wird nur 200 mal in der Sekunde abgetastet. Das für die Kanäle 1A und 1B Gesagte gilt im Prinzip entsprechend auch für die restlichen drei Einschubmodule 2 bis 4. In diesen Einschubmodulen sind entsprechende Kanäle 2A, 3A und 4A bzw. 2B, 3B und 4B vorhanden.

Wie schon angedeutet, sind sämtliche vier Einschübe, was die Kanäle A und B betrifft, gleichartig aufgebaut. Entsprechend der Darstellung der Figur 13 umfassen sie also alle die Signaleingänge E11 und E12. Auf diese Signaleingänge folgen je ein Vorverstärker 197 und 198. Jeder der Vorverstärker hat einen Steuereingang 199 bzw. 200, an dem durch Steuersignal der Verstärkungsgrad reguliert werden kann. Die Einstellung kann anhand von Bit-Kombinationen vorgenommen werden, die zusammen mit einem Konfigurations wort für die Konversion zum signalverarbeitenden Teil auf der Geräteseite zum jeweiligen Modul übertragen werden. Dasselbe gilt z. B. auch für die Durchlaßfrequenzen von Frequenzfiltern 201 und 202, die über entsprechende Steuereingänge 203 bzw. 204 des jeweiligen Filters ebenfalls in Abhängigkeit von entsprechenden Bit-Kombinationen im Konversions-Konfigurationswort einstellbar sind. Auf die Filter 201 und 202 folgen Zustandsmelder 205 und 206, die an Ausgängen 207 und 208 durch entsprechende Bit-Signale den jeweiligen Zustand melden, in dem sich der Modul im betreffenden Kanal befindet. Die Zustandsmelder 205 und 206 umfassen in bekannter Weise z. B. solche Meßglieder, die feststellen, ob die Abnehmer für die einzelnen Signale am Patientenkörper richtig positioniert sind und ob die Signalaufnahme und Übertragung in den Kanälen selbst in Ordnung ist. Diese Daten werden zusammen mit Identifikationssignalen für den jeweiligen Modul und z. B. auch noch zusammen mit weiteren Daten, die beispielsweise über ein Tastenfeld eines Moduls eingegeben wurden, als sogenanntes Hilfswort (Auxiliary Word) vom Modul in Richtung Verarbeitungsteil des Gerätes übertragen. In der Figur 13 ist das Tastenfeld eines Moduls mit der Ziffer 211 schematisch angedeutet. Zur Übermittlung des Hilfswortes dient ein 8 Bit-Schieberegister 212, das an vier Eingängen 213 zu je 1 Bit Hilfsdaten von den Signalgebern 205 und 206 und gegebenenfalls vom Tastenfeld 211 entgegennimmt. Die vier Bits der restlichen vier Eingänge 214 des Schieberegisters 212 formulieren das Identifikationssignal für den jeweiligen Modul. Diese vier Eingänge 214 für das Identifikationssignal sind also in entsprechender Modifikation für jeden Modul unterschiedlich fest verdrahtet. Das Hilfswort (Auxiliary Word) des Schieberegisters 212 eines jeden Einschubmoduls wird zusammen mit den digitalen Meßdaten des Analog-Digital-Konverters 196 über die Sendediode 8 dem zugehörigen Fotoempfänger 9 im Signalverarbeitungsteil des Gerätes übermittelt. Die Signalfolge aus digitalen Daten, die also vom jeweiligen Modul 1 bis 4 in das Gerät gesendet wird, ist in der Figur 15 unten dargestellt. Die Pulse D1 bis D14 sind die digitalen Meßdaten des Analog-Digital-Konverters 196. Die dazwischen gelagerten acht Pulse A1 bis A8 sind die Hilfsdaten ; sie formen also das Hilfswort (Auxiliary Word) und sie beinhalten die Identifikationssignale zur Identifikation des jeweils sendenden Moduls.

Die Pulsfolge, die von einem jeweiligen Lichtsender 10 auf der Seite des Gerätes zu einem Lichtempfänger 11 auf der Seite des jeweiligen Moduls 1 bis 4 übertragen wird und die den Konversionstakt für den jeweiligen Analog-Digital-Konverter 196 vorgibt, ist in der Figur 15 oben angedeutet. Diese Pulsfolge umfaßt pro Konversions-Zyklus insgesamt vierzehn Pulse CL1 bis CL14. Die dazwischen gelagerten Pulse CC1 bis CC8 sind die jeweils 8 Bit, die als Konfigurationswort zur Einstellung der Filter und Verstärker aufgrund von Befehlen dienen, die von der Geräteseite her übermittelt werden. Diese Befehle können durch den jeweiligen Mikroprozessor auf der Geräteseite selbst erzeugt werden. Es können jedoch auch solche Befehle sein, die z. B. durch Tastendruck zuvor vom Einschub her eingegeben und als Bestandteil des Hilfswortes (Auxiliary Word) vom Modul zum Gerät übertragen worden waren und von dort nach entsprechender Umsetzung im jeweiligen Mikroprozessor als Bestandteil des Konfigurationswortes wieder zum Einschub zurückgesendet worden waren. Die Einschubmodule selbst beinhalten keine aktiven Steuerelemente, wie z. B. moduleigene Mikroprozessoren oder dergleichen. Eine Befehlseingabe am Modul über Tasten wirkt also niemals direkt auf den Modul ; sie nimmt vielmehr immer den Umweg über den jeweiligen Mikroprozessor im Signalverarbeitungsteil des Gerätes. Dementsprechend umfaßt also jeder Zyklus von Konversions-pulsen auch entsprechende Pulse eines Konfigurationswortes, mit deren Hilfe im jeweiligen Einschub Befehle ausgeübt werden können.

Wie schon erwähnt, beinhaltet jeder Konversionszyklus insgesamt vierzehn Taktpulse für entsprechend vierzehn Konversionen am Analog-Digital-Konverter 196. Die Taktpulse sind jeweils 2 µs lang und ihr Abstand untereinander beträgt je 12 µs. Im Abstand von 24 µs auf den jeweils vierzehnten Taktpuls CL14 folgt in jedem Zyklus schließlich auch noch ein letzter Puls AZ. Dieser Puls AZ ist ein sogenannter Auto Zero Puls, der über ein Pulsformglied 215 (z. B. Monoflop) den Analog-Digital-Konverter 196 automatisch auf NULL setzt. Berücksichtigt man, daß der jeweils erste Taktpuls CL1 erst 12,5 µs nach Beginn eines Zyklus erzeugt wird und der jeweilige Zyklus erst 12 µs nach Auftreten des AZ Pulses beendet ist, so ergibt sich die Gesamtdauer eines Zyklus zu jeweils 204,5 µs. Jeweils weitere 12,5 µs nach Ende eines Zyklus beginnt dann ein neuer Zyklus, wie in der Figur 15 oben auch schematisch im Pulsdiagramm angedeutet ist.

Im Prinzipschaltbild der Figur 13 gehen die anfallenden Taktimpulse CL1 bis CL14 für den Konversionstakt direkt auf den Analog-Digital-Konverter 196. Die acht Pulse CC1 bis CC8 des Konfigurationswortes werden hingegen in ein 8 Bit-Schieberegister 216 eingeschrieben. Während der Zeitdauer der Einschreibung des jeweiligen Wortes eines Zyklus in das Schieberegister 216 läuft jedoch noch der Konversionstakt des vorhergehenden Zyklus. Das Konversionswort der vorhergehenden Zyklus befindet sich in einem 8 Bit-Schieberegister 217. Während also ein normaler Konversions-Zyklus noch läuft, ist im Schieberegister 216 Information für den nächstfolgenden Zyklus bereits gespeichert. Der jeweilige Modul ist also bereits auf die Forderungen des nächstfolgenden Zyklus eingestellt, obgleich dieser Zyklus noch gar nicht gegonnen hat. Durch diese besonders trickreiche Art der Vorabspeicherung eines Konfigurationswortes mit Befehlsdaten für einen nächstfolgenden Zyklus wird zusätzlich Zeit bei der Konversion gewonnen. Jedes im Schieberegister 216 eingespeicherte Konfigurationswort wird mit Eingang des letzten Impulses CC8 direkt parallel vom zweiten Schieberegister 217 übernommen. Damit steht am Ausgang des zweiten Schieberegisters 217 das Konfigurationswort für den nächstfolgenden Zyklus zur Verfügung.

Das 8 Bit-Schieberegister 217 liefert, wie vorher schon erwähnt, an sechs Ausgängen 220 zu einer Steuereinheit 220a insgesamt sechs Bit Einstellinformation für z. B. Verstärker und Filter. Die beiden restlichen Bits an den Ausgängen 218 und 219 sind Schaltbits für die Abtastschalter 209 und 210 der Kanäle 1A und 1B. Aus diesen wird mittels UND-Glied 220b auch noch ein drittes Schaltsignal für einen Abtastschalter 221 im Hochgeschwindigkeitskanal 1H erzeugt, welcher Kanal 1H ein Filter 222 mit Steuereingang 22a für Hochfrequenzkomponenten umfaßt. Die beiden Bits der Ausgänge 218 und 219 des Schieberegisters 217 legen also fest, mit welcher Rate die einzelnen Kanäle 1H, 1A bis 4A, 1B bis 4B der einzelnen Einschubmodule 1 bis 4 abgetastet werden. Gemäß bevorzugter Ausführung der Erfindung ist die Abtastrate über eine Periodendauer von 4880 µs mit folgender Sequenz festgelegt :

1H, 1A, 1H, 2A, 1H, 3A, 1H, 4A, 1H, 1B, 1H, 2B
1H, 1A, 1H, 2A, 1H, 3A, 1H, 4A, 1H, 3B, 1H, 4B

Der in der Figur 13 dargestellte Analog-Digital-Konverter 196 kann im Prinzip so aufgebaut sein, wie er in der US-PS-3 588 881 beschrieben ist. Das Glied 223 zwischen den Abtastschaltern 209, 210, 221 und dem Analog-Digital-Konverter 196 ist ein übliches Sample and Hold-Glied. Am Ausgang des Analog-Digital-Konverters 196 sitzt eine Puls-Mixstufe 223a.

Die Figur 14 zeigt den prinzipiellen inneren Schaltungsaufbau des Kontroll- und Ankoppelboards 5, so wie es den Einschüben zur Signal- und Energieankoppelung auf der Seite des Gerätes gegenübersteht. Im Vergleich mit Figur 5 ist der vorarbeitende Mikroprozessor (Preprocessor) wieder mit 13 bezeichnet. Der Pfeil 21 führt in Richtung Hauptspeicher 15 (shared memory).

Der Grundtakt für die Pulse eines Konversionszyklus wird von einem Taktgeber 224 (Zähler) geliefert, der vom vorverarbeitenden Prozessor 13 synchronisiert wird. Die Pulse des Taktgebers gehen in ein Steuerwerk 225. In diesem Steuerwerk läuft über entsprechende Pulsschalter der vorstehend beschriebene Abtaktzyklus 1H, 1A, 1H, 2A, etc. Jeder Taktpuls für 1H geht auf eine Taktleitung 226 und auf eine Taktleitung 227. Der Taktpuls der Leitung 226 taktet ein Schieberegister eines Arhythmie-Zusatzes.

In der Leitung 227 steuert derselbe Taktpuls hingegen über die Leitung 228 einen Schalter 229 in die gezeichnete Schaltstellung. Gleichzeitig wird ein Schalter 230 geschlossen und über ein Invertierglied 231 ein Schalter 232 geöffnet. Mit jedem Taktpuls für 1H wird aber auch über eine Steuerleitung 233 ein Abrufsignal für ein Umlaufregister 234 (recirculation register for high speed) erzeugt, in dem ständig Konversionspulse 1H im Umlauf sind. Das Abrufsignal bewirkt die Ausgabe eines Pulses 1H am Ausgang 235 des Umlaufregisters 234. Von dort gelangt der Puls 1H über den zu dieser Zeit geschlossenen Schalter 230 zu einer Überlagerungsstufe 236, die ihn über den Schalter 228 zur Leitung 237 und von dort über die Leitung 238 zur Sendediode 10 des Sendekanals für den obersten Einschubmodul 1 weiterleitet. Die Sendediode 10 sendet den Puls 1H in Richtung Fotoempfänger 11 im Modul 1, der ihn dann als 1H-Tastimpuls einerseits zum Analog-Digital-Wandler 196 und andererseits zu Formation der Schieberegister 216, 217 weiterleitet. Der Puls 1H öffnet außerdem noch einen Schalter 239. Dadurch werden Abtastwerte des 1H-Kanals, die in diesem Augenblick vom Analog-Digital-Konverter des Moduls 1 geliefert werden, nur zum Schieberegister 241 eines Arhythmie-Zusatzes 241 bis 243 geleitet. Von diesem Schieberegister 241 gelangt der jeweilige 1H-meßwert zu einem Digital-Analog-Konverter 242, der ihn zu seinem Analogwert rückwandelt. Der Analogwert kann auf einen Recorder 243 gegeben werden. Das Ergebnis ist dann eine Analogsignalkurve, die über Ereignisse im EKG Auskunft gibt, die übernormal hochfrequent sind.

Die Überlagerungsstufe 236 ist eine Pulsmischstufe, in der den Pulsen 1H in der vorgegebenen Sequenz die restlichen Tastpulse für die Kanäle 1A bis 4A und 1B bis 4B über eine Leitung 244 zugemischt werden. Diesen Pulse werden schließlich über eine Leitung 245 auch noch die acht Pulse CC1 bis CC8 des Konfigurationswortes, das in den jeweiligen Modul rückübertragen werden soll, beigefügt. Die Beimischung erfolgt jeweils über den geschlossenen Schalter 232. Die so gemischte Pulsfolge wird über den außerhalb der Takte 1H in die gestrichelte Stellung gesteuerten Schalter 228 einem Multiplexer 246 zugeführt. Dieser Multiplexer taktet dann, gesteuert vom vorverarbeitenden Prozessor 13, die Sendedioden 10 über Leitungen 246a, 246b, 246c und 246d. Von diesen werden entsprechend der erwünschten Taktfolge Lichtimpulse zu den Fotoempfängern 11 auf der Seite der Einschubmodule 1 bis 4 übermittelt, von wo sie dann schließlich als Konversionstakt zu den Analog-Digital-Konvertern 196 bzw. als Konfigurationswort zu den Schieberegistern 216, 217 gelangen.

Im Fall der Figur 14 ist der Hochgeschwindigkeitkanal für die 1H-Abtastung einem Einschub in oberster Position fest zugeordnet. Relativ hochfrequente Ereignisse treten in der Praxis jedoch höchstens in Verbindung mit der EKG-Messung auf. Damit wird also ein High Speed Signal praktisch nur dann empfangen, wenn der EKG-Modul in die erste Position gesteckt ist. Sitzt in dieser Position hingegen ein Einschub für niederfrequentere Signale, so läuft die Hochgeschwindigkeitsabtastung 1H leer. Wird also High Speed Abtastung gewünscht, so muß der EKG-Einschub in die Position 1 gesteckt werden. Alle andere Positionen sind jedoch beliebig frei wählbar.

Wie in der Figur 15 unten dargestellt ist, empfängt das Gerät von jeweiligen Modul 1 bis 4 Meßwerte D1 bis D14 zusammen mit einem Hilfswort A1 bis A8. In diesem Hilfswort steckt auch das Identifikationssignal für den jeweiligen Einschubmodul. Gemäß der Figur 14 werden nun alle von den Fotoempfängern 9 auf der Seite des Gerätes empfangenen Pulse auf eine Pulsleitung 246c gegeben, von wo sie über den Schalter 239, der zum Zeitpunkt des Anfallens solcher Pulse immer geschlossen ist, zur Leitung 247 gelangen. Von dort werden sie einerseits zum Eingang eines ersten 8 Bit-Schieberegisters 248 weitergereicht. Andererseits gelangen sie über eine Leitung 249 auch auf den Eingang eines zweiten 8 Bit-Schieberegisters 250. Pulse, die im Überlauf das zweite Schieberegister 250 verlassen, werden über eine Leitung 251 in ein drittes Schieberegister 252 eingetaktet, das zwar prinzipiell ebenfalls ein 8 Bit-Schieberegister ist, das jedoch lediglich nur 6 Bit Daten übernimmt.

Die Schieberegister 248, 250 und 252 werden vom Steuerwerk über Taktleitungen 253, 254 und 255 getaktet. Dabei laufen die Schieberegister 250 und 252 im Gegentakt zum Schieberegister 248, das über ein Invertierglied 254 angesteuert wird. Die Leitungen 256, 257 und 258 sind Enable-Steuerleitungen für das jeweilige Schieberegister. Der Gegentakt am Schieberegister 248 hat zur Folge, daß von diesem Register nur solche Daten der anfallenden Pulsfolge eingeschrieben werden, die in Pausen der Meßwertdaten D1 bis D14 anfallen. Solche Daten sind jedoch Daten A1 bis A8 des Hilfswortes (Auxiliary Word). Damit erfaßt also das 8 Bit Schieberegister 248 die Daten des Hilfswortes. Das in Gegentakt arbeitende zweite 8 Bit-Schieberegister 250 taktet hingegen Meßwertdaten. Sobald die ersten acht Einzeldaten angefallen sind, erfolgt Überlauf zum Schieberegister 252. Am Ende eines jeden Konversionszyklus beinhaltet das 8 Bit-Schieberegister 248 also das Hilfswort im Bit-Register 250 die Meßdaten D14 bis D7 und im Register 252 die Meßdaten D6 bis D1 gespeichert sind. Die gespeicherten Daten können nun gruppenweise durch Zwischenspeicher (latches) 259, 260 und 261 zum vorverarbeitenden Prozessor überführt werden. Von dort können sie dem Hauptspeicher zugeleitet und zur weiteren Verarbeitung dem Hauptprozessor überlassen werden. Der Zwischenspeicher 261 und das Schieberegister 252 dienen auch zur Übertragung des Konfigurationswortes vom Preprocessor 13 zur Leitung 245 in solchen Zeiten, in denen im Schieberegister 252 gerade keine Meßdaten gespeichert werden.

Die Figur 16 zeigt das Zusammenspiel zwischen vorverarbeitendem Mikroprozessor (preprocessor), Hauptspeicher (shared memory) und zentralem Mikroprozessor (central processor) eines Gerätes gemäß Figur 5 im detaillierten Prinzipschaltbild.

Wie eingangs schon erwähnt wurde, ist der Vorprozessor 13 nicht DMA-fähig. Der zentrale Mikroprozessor 14 ist hingegen DMA-fähig, d. h. dieser Prozessor steht in fester Zugriffsordnung zum Hauptspeicher 15. Durch besonderen Schaltungstrick wird nun der an sich nicht DMA-fähige vorverarbeitende Mikroprozessor 13 so präpariert, daß er DMA-fähig wird.

Der Trick ist dabei folgender:

Der Vorprozessor 13 erläßt in bestimmten Zeitabständen an den Hauptprozessor 16 eine DMA-Anforderung. Dies geschieht in der Weise, daß am Ausgang des vorverarbeitenden Prozessors 13 eine bestimmte Adresse erscheint. Im vorliegenden Ausführungsbeispiel der Figur 16 besteht diese Adresse z. B. aus drei binären Einsen. Diese Adresse gelangt über eine Adressleitung 263 auf einen Dekoder 264. Dieser Dekoder 264 erkennt die ausgewählte Adresse immer dann, wenn sie anfällt. Er erzeugt daraufhin an seinem Ausgang 265 einen DMA-Puls. Dieser Puls gelangt nun über eine erste Zweigleitung 266 zum zentralen Mikroprozessor 14. Gleichzeitig wird er über eine zweite Zweigleitung 267 auf ein Taktverzögerungsglied 268 gegeben.

Der am zentralen Mikroprozessor 14 eintreffende DMA-Puls löst dort eine DMA-Anforderung aus. Der zentrale Prozessor 14 führt also lediglich noch diejenigen Operationen zu Ende, die er vor dem Eintreffen der DMA-Anforderung in Angriff genommen und noch nicht zu Ende geführt hatte. Der zentrale Mikroprozessor wird also im Augenblick des Eintreffens einer DMA-Anforderung nicht sofort abgeschaltet; bis zur Abschaltung (sämtliche Ausgangspuffer des zentralen Prozessors nehmen einen hochohmigen Widerstandswert ein) vergeht noch eine gewisse Zeit. Während dieser Zeitdauer, während der also der zentrale Prozessor 14 mit dem Hauptspeicher 15 noch in Kommunikation steht, muß gewährleistet sein, daß der vorverarbeitende Mikroprozessor 13 vom Zugriff zum Hauptspeicher 15 noch abgeschaltet bleibt.

Dies geschieht in einfachster Weise durch das Taktverzögerungsglied 268. Bei diesem Taktverzögerungsglied handelt es sich um einen Frequenzuntersetzer, der die Taktfrequenz, mit der der vorverarbeitende Prozessor 13 im normalen Zustand getaktet wird, auf einen gegenüber dem Normalwert sehr viel geringeren Wert herabsetzt. Im vorliegenden Ausführungsbeispiel liegt der Normalwert der Taktfrequenz bei etwa 4 MHz, was einem Pulsabstand der Taktpulse von 0,25 μs entspricht. Wird das Taktverzögerungsglied 268 durch den DMA-Puls angestoßen, so verringert es die Taktfrequenz auf einen Wert von etwa 0,333 MHz. Der Abstand zwischen den Taktpulsen liegt jetz also bei etwa 3 μs.

Der Normalwert der Taktfrequenz wird von einem Taktgeber 269 vorgegeben, der sowohl für den zentralen Prozessor 14 als auch für den vorverarbeitenden Prozessor 13 die Arbeitstakte liefert. Die Taktpulse des Taktgebers 269 gelangen über die Taktleitung 270 zum Frequenzuntersetzer 268. Solange dieser auf Normalbetrieb geschaltet ist, werden die Taktimpulse der Leitung 270 unverzögert über die Leitung 271 dem vorverarbeitenden Prozessor 13 als Arbeitstakte zugeleitet. Liegt hingegen ein DMA-Puls am Frequenzuntersetzer an, so tritt der vorstehend schon beschriebene Arbeitsgang ein ; es werden also die Taktimpulse der Taktleitung 270 in der beschriebenen Weise in der Taktfrequenz untersetzt. Zum vorverarbeitenden Mikroprozessor 13 gelangen dann über die Leitung 271 also nur noch Taktpulse der untersetzten Frequenz. Der vorverarbeitende Prozessor 13 wird hierdurch in seinem Arbeitsablauf verzögert ; er ist trotz bereits ergangener DMA-Anforderung noch nicht kommunikationsbereit. Dies ändert sich in dem Augenblick, in dem vom zentralen Prozessor 14 ein Rückmeldesignal abgegeben wird, mit dem der zentrale Prozessor bestätigt, daß seine eigene Kommunikation mit dem Hauptspeicher 15 abgeschlossen ist.

In der Figur 16 erscheint dieses Rückmeldesignals als DMA-Enable Signal in der Ausgangsleitung 272 des zentralen Prozessors. Von hier aus wird es über einen Leitungszweig 273 einerseits auf das Taktverzögerungsglied 268 gegeben ; andererseits wird es auch gleichzeitig über einen zweiten Leitungsweg 274 den Ausgangspuffern (Buffer) 275, 276 und 277 des vorverarbeitenden Prozessors 13 zugeleitet. Das Enable-Signal bewirkt am Taktverzögerungsglied 268, daß dieses in seine normale Arbeitslage zurückgesetzt wird. Das Glied 268 läßt ab diesem Augenblick also wieder Taktimpulse des Taktgebers 269 mit Normalfrequenz über die Ausgangsleitung 271 zum vorverarbeitenden Prozessor 13 passieren. Der vorverarbeitende Prozessor 13 arbeitet demnach wieder mit seiner normalen Arbeitsfrequenz. Das DMA-Enable Signal schaltet außerdem die vorher hochohmigen Ausgangspuffer 275 bis 277 des vorverarbeitenden Prozessors 13 auf Durchlaß. Damit sind alle Voraussetzungen der DMA-Fähigkeit erfüllt ; der vorverarbeitende Prozessor 13 kann jetzt also über die niederohmigen Ausgangspuffer 275 bis 277 mit dem Hauptspeicher 15 in Kommunikation treten.

Damit ist also ein an sich nicht DMA-fähiger Vorprozessor so geschaltet, als ob er DMA-fähig ist. Der beschriebene Schaltungstrick arbeitet mit einfachsten technischen Mitteln ; eine zusätzliche Software-Unterstützung des Vorprozessors zur Erzeugung der DMA-Anforderung ist nicht nötig. Alle wichtigen Arbeitsgänge werden durchgeführt, ohne daß der Vorprozessor selbst davon Kenntnis nimmt.

Bei der Signalwiedergabe mit Hilfe von dynamischen Wiederholungsspeichern gibt es bekanntlich zwei Möglichkeiten der Signaldarstellung : Bei der ersten Möglichkeit handelt es sich um jene, bei der älteste Information im Speicher jeweils kontinuierlich durch neueste Information ersetzt wird. Am Bildschirm des Darstellungsgerätes führt dies zu einem kontinuierlichen Signalzug aus jeweils ältesten und neuesten Meßwerten. Diese Darstellungsart, die wandernde Signalzüge erzeugt, wird üblicherweise als « paper mode » bezeichnet.

Eine zweite Darstellungsart ist jene des sogenannten « fixed mode ». Hier läuft die einmal in den Speicher eingegebene Signalinformation zyklisch um, ohne daß älteste Information durch neueste Information ersetzt wird. Es entsteht am Bildschirm des Darstellungsgerätes ein eingefrorenes Signalbild. Damit nun am ständig umlaufenden eingefrorenen Bild erkannt werden kann, wo sich im Signalzug Anfang und Ende befinden, wird in üblicher Technik bei Übergang auf « fixed mode » in die Aufzeichnung ein Vertikalbalken eingeblendet, der zusammen mit der Information ständig über den Bildschirm wandert. Der wandernde Vertikalbalken gibt die Grenze zwischen neuer und alter Signalinformation im Speicher an. Er läßt somit erkennen, wo das umlaufende Signal seinen Anfang hat und wo es endet.

Die Figur 17 zeigt die Signal- und Zeichendarstellung der Figur 2 im « fixed mode » mit wanderndem Vertikalbalkens ST. Die Erzeugung eines Vertikalbakens für « fixed mode » bereitet keine Schwierigkeiten für den normalen bekannten Anwendungsfall, daß nämlich die vom Bildwiederholungsspeicher zyklisch ausgegebene Information direkt auf das eigene Darstellungsgerät gegeben wird.

Probleme entstehen jedoch bei Anwendung des « fixed mode » auf Composite Video Darstellung. Eine direkte Übertragung eines Dunkeltastimpulses, der Grundlage für eine Balkenanzeige ist, zwischen einzelnen Geräten einer Ketten- oder Sternkonfiguration ist nicht möglich.

Die Figur 18 zeigt ein Prinzip, wie besonders trickreich und ohne großen zusätzlichen Schaltungsaufwand auch für Composite Video die Anwendung der Balkendarstellung möglich ist, so daß auch hier eine wahlweise Darstellung im « paper mode » oder im « fixed mode » erfolgen kann. Das Prinzipschaltbild der Figur 18 ist dem Prinzipschaltbild der Figur 6 sehr ähnlich. Es ist lediglich noch durch solche Bauelemente ergänzt, die zur Balkenerzeugung notwendig sind. Demgemäß besitzt also das Gerät des Prinzipschaltbildes der Figur 18 zwei Wahltasten 278 und 279. Mittels der Taste 278 kann der Betriebszustand « paper mode » gewählt werden ; mit der Taste 279 kann auf « fixed mode » geschaltet werden.

Das Drücken einer Wähltaste 278 oder 279 wird vom zentralen Mikroprozessor 14 als Wahl einer bestimmten Betriebsart registriert.

In Normalfall steht der Schalter 280 am Eingang des Adressrechners 25 in der Schaltstellung « paper mode ». Mit Betätigung der Taste 278 ist die Betriebsart « paper mode » also bereits angewählt. Die Schaltung arbeitet so, wie es in den voraugsgegangenen Passagen geschildert wurde.

Wird hingegen die Wähltaste 279 gedrückt, so erzeugt der zentrale Mikroprozessor 14 ein Umschaltsignal für den Schalter 280. Der Schalter 280 wird also in die Schaltstellung « fixed mode » gesteuert.

Damit ergibt sich im Unterschied zu « paper mode » der folgende Funktionsablauf :

Der Zugang von neuer Meßwert-Information zum Bildwiederholungsspeicher 24 wird vom zentralen Mikroprozessor 14 gesperrt. Seitens des Adressrechners 25 wird jedoch ein Adress-Streifen aus z. B. insgesamt 16 × 8 EINSEN erzeugt.

Dieser Adress-Streifen wird nun vom Adressrechner an jene Stelle im Bildwiederholungsspeicher 24 eingeschrieben, an der im Speicher älteste Signalinformation auf jeweils neueste Signalinformation trifft. Der Adress-Streifen 281 aus binären EINSEN grenzt also im Speicher den Signalanfang gegenüber dem Signalende ab.

Der Adress-Streifen 281 auf 16 × 8 EINSEN wird nun zusammen mit der ständig umlaufenden gespeicherten Signalinformation zyklisch vom Bildwiederholungsspeicher 24 über das zentrale Schieberegister 23 in Richtung Darstellungsgerät ausgegeben. Dabei gelangen die EINSEN auf ein logisches Gatter 282, das dem z-Helltastgenerator des x, y, z-Oszilloskops vorgeschaltet ist. Das logische Gatter erkennt das Vorliegen eines Adress-Streifens aus EINSEN ; es erzeugt daraufhin während der Zeitdauer des Anfallens der EINSEN ein Dunkeltastsignal für den z-Helltastgenerator 49, das diesem über den in der dargestellten Schaltstellung befindlichen Schalter 284 zugeleitet wird. Der Helltastgenerator 49 bleibt also während der Zeitdauer des Dunkeltastsignals ausgeschaltet.

Jeder Adress-Streifen 281 aus EINSEN, der vom Bildwiederholungsspeicher 24 über das Schieberegister 23 ausgegeben wird, trifft gleichzeitig aber auch auf den Eingang des Analog-Digital-Konverters 47. Der Analog-Digital-Konverter 47 erzeugt daraufhin einen Ausgangsimpuls, dessen Amplitude wegen der nur aus EINSEN bestehenden Eingangsinformation sehr viel höher ist als normale anfallende Signalamplituden. Diese hochamplitudige Ausgangsimpuls gelangt auf die Vertikalablenkeinrichtung 48 des Oszilloskops 28. Er führt dort zu einer Übersteuerung. Der Elektronenstrahl des Oszilloskops wird sehr rasch und sehr weit über den Bildschirm ausgelenkt. Die Flanken des Impulses sind wegen der sehr raschen Strahlablenkung nicht sichtbar. Der gleichzeitig eintreffende Dunkeltastimpuls führt zur Dunkeltastung in den Grenzen des Ablenkimpulses. Am Bildschirm des Oszilloskops wird also ein Dunkelstreifen für « fixed mode » sichtbar. Dieser Dunkelstreifen wandert zusammen mit der ständig umlaufenden Signalinformation des eingefrorenen Bildes über den Bildschirm des Oszilloskops. Es ist damit also immer eine eindeutige visuelle Aussage möglich darüber, wo sich im wandernden Bild gerade der Anfang der abgebildeten Signale befindet und wo das Ende.

Der am Ausgang des Analog-Digital-Konverters 47 anfallende Ausgangsimpuls sehr hoher Amplitude gelangt jedoch nicht nur zum geräteeigenen Oszilloskop ; er wird auch immer gleichzeitig über den Leitungsanschluß 53 als Bestandteil des normalen Composite Video Signals dem Composite Video Output 30 des Gerätes zugeführt. Von dort gelangt er auf den Composite Video Bus, sofern der Ausgang 30 am Bus angeschaltet ist.

Was den Empfangsteil eines Gerätes betrifft, so ist dort folgende Abänderung vorgesehen :

Im Empfangsteil ist an der Leitung 59, die vom Composite Video Input 31 zum Schalter 56 für das Analogsignal führt, ein Komparator 283 angeschaltet. Dieser Komparator 283 besitzt einen Referenzspannungseingang, an dem eine Referenzspannung $U_{ref}$ liegt. Ist also ein beliebiges Gerät auf « fixed mode » geschaltet und soll dieses Gerät Composite Video Signale eines anderen Gerätes empfangen, das ebenfalls im « fixed mode » arbeitet, so werden der Composite Video Output des sendenden Gerätes und der Composite Video Input des empfangenden Gerätes miteinander verbunden. Dies kann bei Kettenschaltung der Geräte über den gemeinsamen Composite Video Bus oder bei Sternschaltung der Geräte über den zentralen Composite Video Bus der Zentrale geschehen.

Das empfangende Gerät erhält jetzt ein Composite Video Signal, das im Analogteil einen Ausgangsimpuls hoher Amplitude als Streifensignal enthält. Das Auftreten des hochamplitudigen Impulses wird vom Komparator 283 als schwellwertüberschreitendes Ereignis erkannt. Der Komparator 283 erzeugt daraufhin während der Zeitdauer des Schwellwertüberschreitung ein Ausgangssignal. Dieses Ausgangssignal dient jetzt speziell als Autastsignal für den Helltastgenerator 49. Es wird dem Helltastgenerator 49 über den jetzt in die gestrichelte Schaltstellung gesteuerten Schalter 284 zugeführt. Der Helltastgenerator 49 bewirkt wieder Strahldunkeltastung während der Auftrittsdauer des fremden Streifenpulses. Am Bildschirm des empfangenden Gerätes erscheint jetzt also das « fixed mode »-Signalbild des fremden sendenden Gerätes mit eingeblendetem Vertikalbalken als Grenzangabe für Signalanfang und Signalende.

In den Figuren 1 bis 18 ist lediglich ein bevorzugtes Ausführungsbeispiel der Erfindung beschrieben. Es versteht sich von selbst, daß die Erfindung nicht auf dieses Ausführungsbeispiel beschränkt ist. Vielmehr gibt es beliebige Modifikationen des Ausführungsbeispieles, die alle mit unter den Schutz der Erfindung fallen sollen.

**Patentansprüche**

1. Geräteanordnung mit einer vorgegebenen Zahl von Überwachungsgeräten (84, 85) und wenigstens einer Gerätezentrale (83), die ein zentrales Darstellungsgerät (96) mit diesem zugeordneten Zwischenspeicher (97) und einem zentralen Multiplexer (99) enthält, wobei jedes Überwachungsgerät (84, 85) wenigstens einen Signalgeber (88, 89) umfaßt, dessen Signale an einem dem betreffenden Überwachungsgerät eigenen Darstellungsgerät (86, 87) als gerätespezifisches Bild darstellbar sind, und wobei die Gerätezentrale (83) mit den Signalgebern (88, 89) der Überwachungsgeräte (84, 85) durch eine Anwähleinrichtung verbindbar ist derart, daß die Gerätezentrale (83) von dem Signalgeber (88, 89) eines beliebig angewählten Überwachungsgerätes (84, 85) Signale zur Darstellung auf dem zentralen Darstellungsgerät (96) übernimmt, dadurch gekennzeichnet, daß zwischen den Signalgebern (88, 89) der Überwachungsgeräte (84, 85) und dem zentralen Darstellungsgerät (96) der Gerätezentrale (83) der zentrale Multiplexer (99) eingeschaltet ist, der für das Ausgangssignal (CVBI bis CVBn) eines jeden Signalgebers (88, 89) eines Überwachungsgerätes (84, 85) einen separaten Signaleingang (90, 91, 92) besitzt und der eine erste und eine zweite Zahl von Ausgängen (102, 103, 104, 105, 111) aufweist, von denen die erste Zahl an Ausgängen (111) direkt in Richtung auf das zentrale Darstellungsgerät (96) geht, während die zweite Zahl an Ausgängen (102 bis 105) zum Eingang des Zwischenspeichers (97) geht, dessen Signalausgang in Richtung auf das zentrale Darstellungsgerät (96) führt, und daß der zentrale Multiplexer (99) von einer Steuereinheit (101) so gesteuert ist, daß er der ersten Zahl von Ausgängen (111) im Zeitmultiplex jeweils nur solche Signaldaten (CVB) zuführt, die von Signalgeber (z. B. 88) eines einzelnen ausgewählten Überwachungsgerätes (z. B. 84) stammen, während er der zweiten Zahl von Ausgängen (102 bis 105) nur die jeweils ältesten Signaldaten (A, B, C, D) aller Signalgeber (88, 89) zuführt,wobei die jeweils ältesten Signaldaten im Zwischenspeicher (97) gespeichert werden und von dort in beliebigen Signalkombinationen (CVC) zur Aufzeichnung als Mischbild auf dem zentralen darstellungsgerät (96) abrufbar sind.

2. Geräteanordnung nach Anspruch 1, dadurch gekennzeichnet, daß die Gerätezentrale (83) in der Verbindungsleitung zwischen Darstellungsgerät (96) und Zwischenspeicher (97) einen speziellen composite video Zentralausgang umfaßt, zu dem dieselben Signale (CVC) von Zwischenspeicher (97) ausgegeben werden, die gleichzeitig vom Zwischenspeicher (97) zum zentralen Darstellungsgerät zum Aufbau eines Mischbildes abgerufen werden.

3. Geräteanordnung nach Anspruch 2, dadurch gekennzeichnet, daß vom speziellen composite video Zentralausgang ein Signalbus (132) zu den Überwachungsgeräten (84, 85) zurückgeführt ist und daß vorzugsweise jedes Überwachungsgerät (84, 85) in der Signalleitung zwischen dem geräteeigenen Signalgeber (88, 89) und dem geräteeigenen Darstellungsgerät (86, 87) ein Schaltglied (133, 134) aufweist, das aus einer ersten Schaltstellung, in der es das Darstellungsgerät zur Darstellung des geräteeigenen Signalbildes mit dem geräteeigenen Signalgeber verbindet, in eine zweite Schaltstellung steuerbar ist, in der es das Darstellungsgerät mit dem vom Zentralgerät (83) kommenden Signalbus (132) verbindet, so daß ein beliebiges Überwachungsgerät also aus dem Signalbus das zentrale Mischbild des zentralen Darstellungsgerätes (96) zur Darstellung auf dem geräteeigenen Darstellungsgerät übernehmen kann.

4. Geräteanordnung nach einem der Ansprüche 1 bis 3, dadurch gekennzeichnet, daß die Gerätezentrale (83) auch noch einen Alarm-Multiplexer (100) umfaßt, der Alarmeingänge aufweist, die über Alarmleitungen (93, 94, 95) mit Alarmgebern (z. B. 32) auf der Seite der Überwachungsgeräte (84, 85) verbunden sind, welche Alarmgeber ein Alarmsignal erzeugen, wenn ein Signal einen kritischen Zustand signalisiert, und welcher Alarm-Multiplexer (100) im Zeitmultiplex die Alarmeingänge auf das Vorliegen eines Alarmsignals abfragt, und beim Vorliegen eines Alarmsignals dieses zu einem Mikroprozessor (98) weiterleitet, der daraufhin das zentrale Darstellungsgerät (96) in dem Sinne steuert, daß die alarmbehaftete Signalspur des alarmgebenden Überwachunggerätes im zentralen Signalbild als Signalspur in einem ausgewählten Darstellungskanal erscheint.

5. Geräteanordnung nach Anspruch 4, dadurch gekennzeichnet, daß bei mehreren Alarmzuständen in unterschiedlichen Überwachungsgeräten die betreffenden alarmbehafteten Signalspuren in der Reihenfolge des Auftretens der Alarme in vorgebbarer Kanal-Reihenfolge in unterschiedliche Darstellungskanäle übernommen werden.

6. Geräteanordnung nach Anspruch 5, dadurch gekennzeichnet, daß eine alarmgebende Signalspur vom Mikroprozessor (98) so in eine Signalbild-Darstellung am zentralen Darstellungsgerät (96) geschoben wird, daß sie im höchstnumerierten Kanal erscheint.

7. Geräteanordnung nach Anspruch 6, dadurch gekennzeichnet, daß bei einem zentralen Darstellungsgerät (96) mit vier Kanälen eine alarmbehaftete Signalspur in den vierten Kanal geschoben wird.

8. Geräteanordnung nach Anspruch 6 oder 7, dadurch gekennzeichnet, daß bei Auftreten weiterer Alarme die betreffenden alarmbehafteten Signalspuren in Kanäle mit den jeweils nächstniedrigen Kanalnummern geschoben werden.

9. Geräteanordnung nach einem der Ansprüche 1 bis 8, dadurch gekennzeichnet, daß der Multiplexer (99) für die Signaldaten auch noch einen Ausgang (106) zur Übermittlung von Adress-Signalen (CH), die in den Eingangssignalen (CVBI bis CVBn) enthalten sind, umfaßt, welche Adress-Signale zur Ansteuerung eines Zeichengenerators (45) dienen, der in Verbindung mit einem schnellen y-Ablenkraster ($Y_R$) eines y-Rampenspannungsgenerators (46) und z-Helltastimpulsen eines z-Helltastgenerators (49) alphanumerische Zeichen, z. B. Buchstaben und Zahlen, in das Signalbild einblendet.

10. Geräteanordnung nach Anspruch 9, dadurch gekennzeichnet, daß der Adress-Ausgang (106) des Multiplexers (99) zum Zwischenspeicher (101) führt, von dem gesteuert durch einen Mikroprozessor (98) die Adressweiterleitung zum zentralen Darstellungsgerät (96) erfolgt.

11. Geräteanordnung nach einem der Ansprüche 4 bis 10, dadurch gekennzeichnet, daß im Alarmfall am zentralen Darstellungsgerät (96) neben der alarmgebenden Signalspur über den Mikroprozessor (98) zusätzlich zu dieser Signalspur auch noch der Ort der Alarmgebung als alphanumerisches Zeichen einblendbar ist.

12. Geräteanordnung nach Anspruch 11, dadurch gekennzeichnet, daß bei elektromedizinischen Überwachungsgeräten zusammen mit der alarmgebenden Signalspur die Nummer des Bettes als vorzugsweise blinkendes Zeichen für den Alarmort am zentralen Darstellungsgerät einblendbar ist.

13. Geräteanordnung nach einem der Ansprüche 1 bis 12, dadurch gekennzeichnet, daß der Multiplexer (99) für die Signaldaten auch noch eine vorgebbare Zahl von Ausgängen (113, 114, 115) für den Anschluß von Signal-Recordern (116, 117, 118) umfaßt.

14. Geräteanordnung nach Anspruch 13, dadurch gekennzeichnet, daß die Recorder (116, 117, 118) auch einen Anschluß (132) für die Signale (CVC) eines Mischbildes aufweisen.

15. Geräteanordnung nach Anspruch 13 oder 14, dadurch gekennzeichnet, daß wenigstens ein Recorder (z. B. 116) als Trendrecorder zur Aufzeichnung von Trenddaten ausgebildet ist.

16. Geräteanordnung nach einem der Ansprüche 1 bis 15, dadurch gekennzeichnet, daß der Zwischenspeicher (97) der Gerätezentrale (83) ein Bildwiederholungsspeicher ist, der von den ebenfalls als Bildwiederholungsspeichern ausgebildeten Signalgebern (88, 89) der Überwachungsgeräte (84, 85) immer solche Signalwerte erhält, die gerade als älteste Werte durch neueste Werte ersetzt werden.

## Claims

1. A device arrangement with a predetermined number of monitoring devices (84, 85) and at least one device central control (83) which includes a central representation device (96) with an assigned intermediate store (97) and a central multiplexer (99), where each monitoring device (84, 85) comprises at least one signal generator (88, 89), the signals of which can be represented, as a device-specific image, on a representation device (86, 87) assigned to the monitoring device in question, and where the device central control (83) can be connected to the signal generators (88, 89) of the monitoring devices (84, 85) by means of a selection device, in such manner that the device central control (83) receives, from the signal generator (88, 89) of a random selected monitoring device (84, 85), signals for representation on the central representation device (96), characterised in that between the signal generators (88, 89) of the monitoring devices (84, 85) and the central representation device (96) of the central control (83), there is connected the central multiplexer (99) which has a separate signal input (90, 91, 92) for the output signal (CVBl to CVBn) of each signal generator (88, 89) of a monitoring device (84, 85), and which has a first and second number of outputs (102, 103, 104, 105, 111), of which the first number of outputs (111) proceeds directly in the direction of the central representation device (96), where the second number of outputs (102 to 105) proceeds to the input of the intermediate store (97) whose signal output leads in the direction of the central representation device (96), and that the central multiplexer (99) is controlled by means of a control unit (101) in such manner that it supplies the first number of outputs (111), in time division multiplex, only with those items of signal data (CVB) which emanate from the signal generator (e. g. 88) of an individual, selected monitoring device (e. g. 84), whereas it supplies the second number of outputs (102 to 105) only with the earliest items of signal data (A, B, C, D) of all the signal generators (99, 89), where the earliest items of signal data are stored in the intermediate store (97) from where they can be called up, in arbitrary signal combinations (CVC), to be recorded as a mixed image on the central representation device (96).

2. A device arrangement as claimed in claim 1, characterised in that in the connection line between the representation device (96) and the intermediate store (97) the device central control (83) comprises a special composite video central output to which the same signals (CVC) are output from the intermediate store (97) as are simultaneously called up from the intermediate store (97) to the central representation device for the construction of a mixed image.

3. A device arrangement as claimed in claim 2, characterised in that from the special composite video central output a signal bus (132) leads back to the monitoring devices (84, 85), and that preferably, in the signal line between the device-individual signal generator (88, 89) and the device-individual representation device (86, 87), each monitoring device (84, 85) includes a switching element (133, 134) which can be controlled from a first switching position, in which it connects the representation device for the representation of the device-individual signal image to the device-individual signal generator, into a second switching position in which it connects the representation device to the signal bus (132) emanating from the central device (83), so that an arbitrary monitoring device can thus receive from the signal bus the central mixed image of the central representation device (96) for representation on the device-individual representation device.

4. A device arrangement as claimed in one of the claims 1 to 3, characterised in that the device central control (83) also comprises an alarm multiplexer (100) which has alarm inputs which are connected via alarms lines (93, 94, 95) to alarm generators (e. g. 32) on the side of the monitoring devices (84, 85), which alarm generators generate an alarm signal when a signal signals a critical state, and which alarm multipexer (100) interrogates the alarm inputs in time division multiplex as regards the existence of an alarm signal, and on the existence of an alarm signal forwards this to a microprocessor (98) which subsequently controls the central representation device (96) in such manner that the alarm-bearing signal track of the alarm-generating monitoring device appears in the central signal image as a signal track in a selected representation channel.

5. A device arrangement as claimed in claim 4, characterised in that in the event of a plurality of alarm states in different monitoring devices, the alarm-bearing signal tracks in question are transferred, in the sequence of the occurrence of the alarms, in a predeterminable channel sequence into different representation channels.

6. A device arrangement as claimed in claim 5, characterised in that an alarm-generating signal track is forwarded from the microprocessor (98) into a signal image representation on the central representation device (96) in such manner that it appears in the highest numbered channel.

7. A device arrangement as claimed in claim 6, characterised in that in the case of a central representation device (96) having four channels, an alarm-bearing signal track is forwarded into the fourth channel.

8. A device arrangement as claimed in claims 6 or 7, characterised in that on the occurrence of further alarms, the alarm-bearing signal tracks in question are forwarded into channels having the next-lower channel numbers.

9. A device arrangement as claimed in one of the claims 1 to 8, characterised in that the multiplexer (99) for the items of signal data also has an output (106) for the transfer of address-signals (CH) which are included in the input signals (CVBI to CVBn), which address-signals serve to drive a character generator (45) which, in combination with a high-speed y-deflecting matrix ($Y_R$) of a y-ramp voltage generator (46) and z-unblanking pulses of a z-unblanking generator (49), gates alpha-numerical characters, e. g. letters and numbers, into the signal image.

10. A device arrangement as claimed in claim 9, characterised in that the address-output (106) of the multiplexer (99) leads to the intermediate store (101), from which, under the control of a microprocessor (98), the addresses are forwarded to the central representation device (96).

11. A device arrangement as claimed in one of the claims 4 to 10, characterised in that in the event of an alarm on the central representation device (96) in addition to the alarm-generating signal track, via the microprocessor (98), in addition to the signal track the location of the alarm generation can also be gated-in as an alphanumerical character.

12. A device arrangement as claimed in claim 11, characterised in that in electromedicinal monitoring devices, together with the alarm-generating signal track, the number of the bed can also be gated-in on the central representation device as a preferably flashing symbol of the alarm location.

13. A device arrangement as claimed in one of the claims 1 to 12, characterised in that the multiplexer (99) for the items of signal data also has a predeterminable number of outputs (113, 114, 115) for the connection of signal recorders (116, 117, 118)

14. A device arrangement as claimed in claim 13, characterised in that the recorders (116, 117, 118) also have a terminal (132) for the signals (CVC) of a mixed image.

15. A device arrangement as claimed in claims 13 or 14, characterised in that at least one recorder (e. g. 116) is designed as a trend recorder for the recording of items of trend data.

16. A device arrangement as claimed in one of the claims 1 to 15, characterised in that the intermediate store (97) of the device central control (83) comprises an image repetition store which always receives from the signal generators (88, 89) — which likewise comprise image repetition stores — of the monitoring devices (84, 85) signal values which are currently being replaced, as earliest values, by most recent values.

**Revendications**

1. Ensemble d'appareils comportant un nombre prédéterminé d'appareils de contrôle (84, 85) et au moins un dispositif central (83) qui contient un appareil central de représentation (96) ainsi qu'une mémoire intermédiaire (97), associée à cet appareil, et un multiplexeur central (99), et dans lequel chaque appareil de contrôle (84, 85) comporte au moins un générateur de signaux (88, 89), dont les signaux peuvent être représentés, sous la forme d'une image spécifique à l'appareil, sur un appareil de représentation (86, 87) propre à l'appareil de contrôle concerné, et dans lequel le dispositif central (83) prévu pour les appareils peut être relié par un dispositif de sélection aux générateurs de signaux (88, 89) des appareils de contrôle (84, 85) de telle sorte que ledit dispositif central (83) reçoit, de la part du générateur de signaux (88, 89) d'un appareil de contrôle (84, 85), sélectionné d'une manière quelconque, des signaux en vue de leur représentation sur l'appareil central de représentation (96), caractérisé par le fait qu'entre les générateurs de signaux (88, 89) des appareils de contrôle (84, 85) et l'appareil central de représentation (96) du dispositif central (83) se trouve branché le multiplexeur central (99) qui possède une entrée séparée de signaux (90, 91, 92) pour le signal de sortie (CVBI à CVBn) de chaque générateur de signaux (88, 89) d'un appareil de contrôle (84, 85) et qui possède un premier et un second nombres de sorties (102, 103, 104, 105, 111), parmi lesquels le premier nombre de sorties (111) partent directement en direction de l'appareil central de représentation (96), tandis que le second nombre de sorties (102 à 105) aboutissent à l'entrée de la mémoire intermédiaire (97) dont la sortie des signaux part en direction de l'appareil central de représentation (96), et que le multiplexeur central (99) est commandé par une unité de commande (101) de telle sorte qu'il envoie au premier nombre de sorties (111), selon un multiplexage temporel, uniquement les données de signaux (CVB), qui proviennent du générateur de signaux (par exemple 88) d'un appareil individuel choisi de contrôle (par exemple 84), tandis qu'il envoie au second nombre de sorties (102 à 105) uniquement les données respectivement les plus anciennes de signaux (A, B, C, D) de tous les générateurs de signaux (88, 89), les données respectivement les plus anciennes de signaux étant mémorisées dans la mémoire intermédiaire (97) et pouvant être appelées, à partir de là, selon des combinaisons quelconques de signaux (CVC), en vue de leur affichage sous la forme d'une image mixte sur l'appareil central de représentation (96).

2. Ensemble d'appareils suivant la revendication 1, caractérisé par le fait que le dispositif central (83) comporte dans la ligne de liaison entre l'appareil de représentation (96) et la mémoire intermédiaire (97) une sortie centrale particulière du signal vidéo composite, à laquelle la mémoire intermédiaire (97) délivre les signaux (CVC) qui sont appelés simultanément de la mémoire intermédiaire (97) en direction de l'appareil central de représentation afin de former une image mixte.

3. Ensemble d'appareils suivant la revendication 2, caractérisé par le fait qu'un bus de transmission de signaux (132) est raccordé, par réaction, à partir de la sortie centrale particulière du signal vidéo composite, aux appareils de contrôle (84, 85) et que de préférence chaque appareil de contrôle (84, 85) possède, dans la ligne de transmission de signaux entre le générateur de signaux (88, 89), qui est propre à l'appareil, et l'appareil de représentation (86, 87) qui est propre à l'appareil, un organe de commutation (133, 134) qui peut être amené, sur commande, d'une première position de commutation dans laquelle il relie l'appareil de représentation servant à représenter l'image du signal, propre à l'appareil, au générateur de signaux propre à l'appareil, dans une seconde position de commutation dans laquelle il relie l'appareil de représentation au bus de transmission de signaux (132) arrivant du dispositif central (83), de sorte que n'importe quel appareil de contrôle peut par conséquent prendre en charge, à partir du bus de transmission de signaux, l'image mixte centrale de l'appareil central de représentation (96) en vue de sa représentation sur l'appareil de représentation propre à l'appareil.

4. Ensemble d'appareils suivant l'une des revendications 1 à 3, caractérisé par le fait que le dispositif central (83) comporte en outre un multiplexeur d'alarme (100), qui possède des entrées d'alarme qui sont reliées par l'intermédiaire de lignes d'alarme (93, 94, 95) à des générateurs d'alarme (par exemple 32) prévus du côté des appareils de contrôle (84, 85), mais qui produisent un signal d'alarme lorsqu'un signal indique un état critique, et que le multiplexeur d'alarme (100) interroge les entrées d'alarme du point de vue de la présence éventuelle d'un signal d'alarme et, dans le cas où un tel signal est présent, le retransmet à un microprocesseur (98) qui commande alors le dispositif central de représentation (96) de manière que la trace du signal, affectée d'une alarme, de l'appareil de contrôle délivrant l'alarme apparaisse dans l'image centrale du signal sous la forme d'une trace de signaux dans un canal de représentation sélectionné.

5. Ensemble d'appareils suivant la revendication 4, caractérisé par le fait que dans le cas de plusieurs états d'alarme dans différents appareils de contrôle, les traces considérées de signal, affectées une alarme, sont transférées, conformément à la succession d'apparitions des alarmes et dans une succession pouvant être prédéterminée de canaux, dans des canaux différents de représentation.

6. Ensemble d'appareils suivant la revendication 5, caractérisé par le fait qu'une trace de signal, produisant une alarme, est transférée depuis le microprocesseur (98) dans une représentation imagée du signal dans l'appareil central de représentation (96) de telle sorte qu'il apparaît dans le canal possédant le numéro le plus élevé.

7. Ensemble d'appareils suivant la revendication 6, caractérisé par le fait que dans le cas d'un dispositif central de représentation (94) comportant quatre canaux, une trace de signaux affectée d'une alarme est décalée dans le quatrième canal.

8. Ensemble d'appareils suivant la revendication 6 ou 7, caractérisé par le fait que lors de l'apparition d'autres alarmes, les traces de signaux concernées, affectées d'une alarme, sont décalées dans des canaux possédant les numéros de rang directement inférieur.

9. Ensemble de dispositifs suivant l'une des revendications 1 à 8, caractérisé par le fait que le multiplexeur (99) prévu pour les données de signaux contient en outre une sortie (106) servant à la retransmission de signaux d'adresses (CH), qui sont contenus dans les signaux d'entrée (CVBI à CVBn) et qui servent à commander un générateur de caractères (45), qui insère, dans l'image du signal, des caractères alphanumériques, par exemple des lettres et des chiffres, en liaison avec un réseau de balayage y rapide ($Y_R$) d'un générateur (46) de tension en rampe y, avec des impulsions de balayage avec faisceau allumé z d'un générateur (49) d'impulsions de balayage avec faisceau allumé z.

10. Ensemble d'appareils suivant la revendication 9, caractérisé par le fait que la sortie d'adresses (106) du multiplexeur (99) est reliée à la mémoire intermédiaire (101), à partir de laquelle la retransmission des adresses s'effectue en direction de l'appareil central de représentation (96), sous la commande d'un microprocesseur (98).

11. Ensemble d'appareils suivant l'une des revendications 4 à 10, caractérisé par le fait que dans le cas d'une alarme dans l'appareil central de représentation (96), non seulement la trace de signaux indiquant une alarme, mais également, en plus de cette trace de signaux le lieu de la délivrance de l'alarme peuvent être insérés sous la forme d'un caractère alphanumérique par l'intermédiaire du microprocesseur (98).

12. Ensemble d'appareils suivant la revendication 11, caractérisé par le fait que dans le cas d'appareils de contrôle électromédicaux, outre la trace de signaux délivrant une alarme, le numéro du lit peut être inséré sous la forme d'un caractère de préférence clignotant pour l'indication du lieu d'alarme dans l'appareil central de représentation.

13. Ensemble de dispositifs suivant l'une des revendications 1 à 12, caractérisé par le fait que le multiplexeur (99) prévu pour les données de signaux comporte en outre un nombre pouvant être prédéterminé de sorties (113, 114, 115) pour le raccordement d'enregistreurs de signaux (116, 117, 118).

14. Ensemble d'appareils suivant la revendication 13, caractérisé par le fait que les enregistreurs (116, 117, 118) comportent également un raccord (132) pour les signaux (CVC) d'une image mixte.

15. Ensemble d'appareils suivant la revendication 13 ou 14, caractérisé par le fait qu'au moins un enregistreur (par exemple 116) est réalisé sous la forme d'un enregistreur de tendance servant à représenter des données de tendance.

16. Ensemble d'appareils suivant l'une des revendications 1 à 15, caractérisé par le fait que la mémoire intermédiaire (97) du dispositif central (83) est une mémoire de répétition d'images qui reçoit toujours, par des générateurs de signaux (88, 89), réalisés également sous la forme de mémoires de répétition d'images, des apareils de contrôle (84, 85), des valeurs de signaux, qui, précisément, en tant que valeurs les plus anciennes, sont remplacées par des valeurs plus récentes.

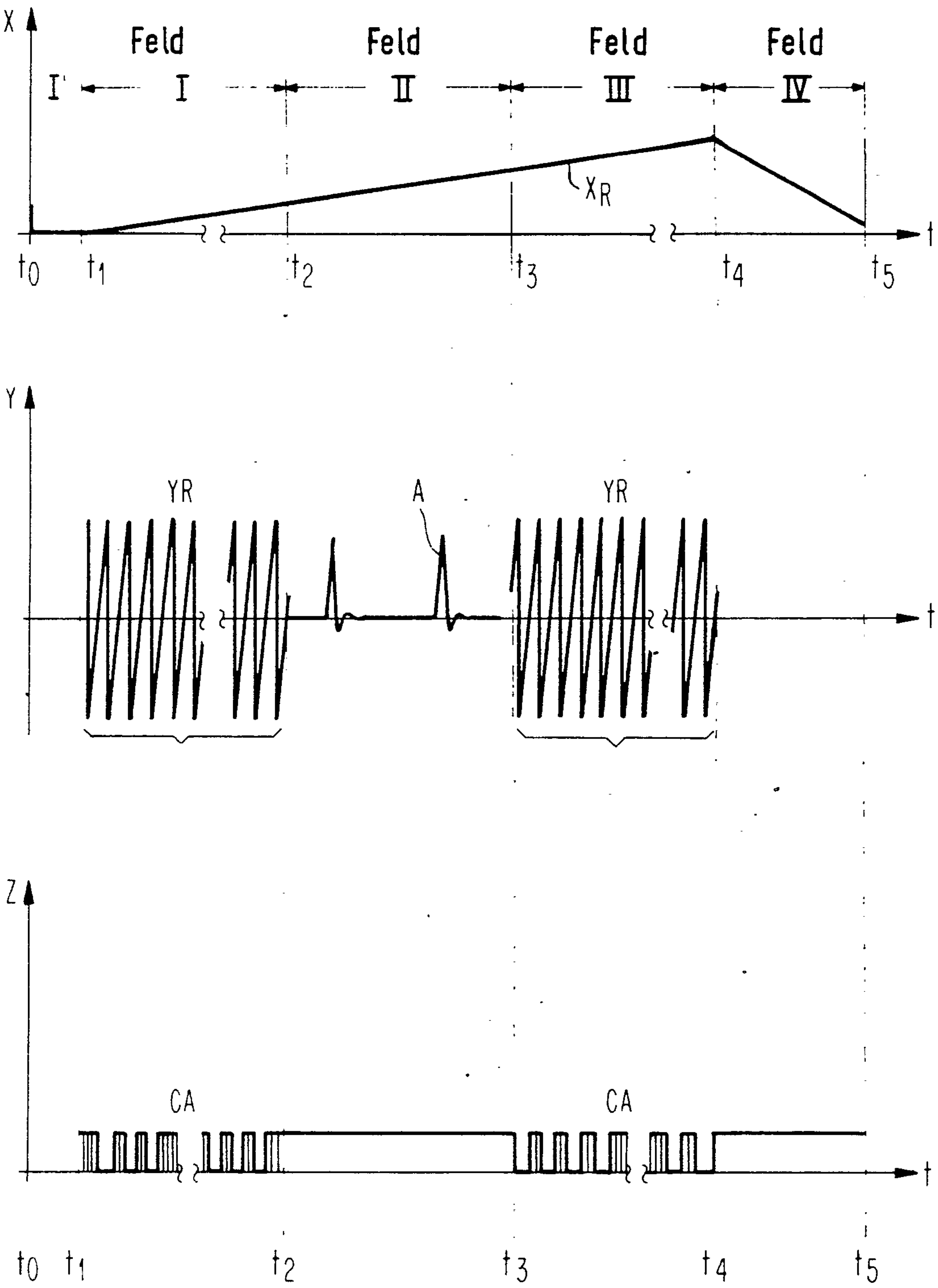
X
Feld
I'
I
Feld
II
Feld
III
Feld
IV
XR
t0
t1
t2
t3
t4
t5
t
Y
YR
A
YR
t
Z
CA
CA
t
t0
t1
t2
t3
t4
t5

FIG. 1

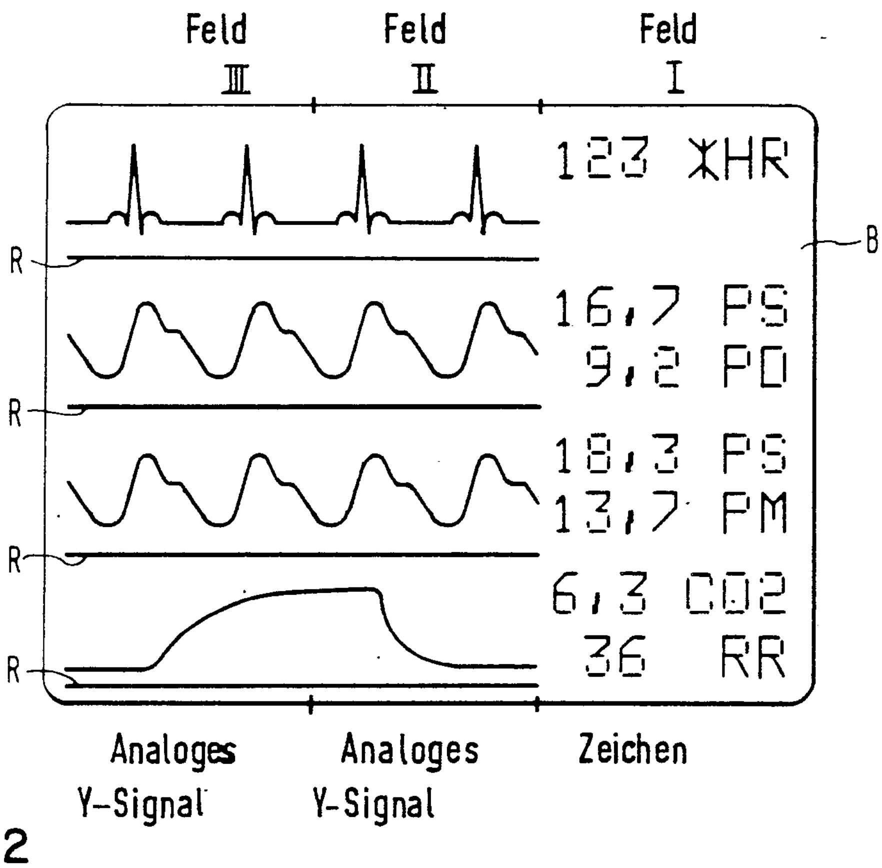
Feld
III
Feld
II
Feld
I
123 HR
B
R
16,7 PS
9,2 PD
R
18,3 PS
13,7 PM
R
6,3 CO2
36 RR
R
Analoges
Y-Signal
Analoges
Y-Signal
Zeichen

FIG. 2

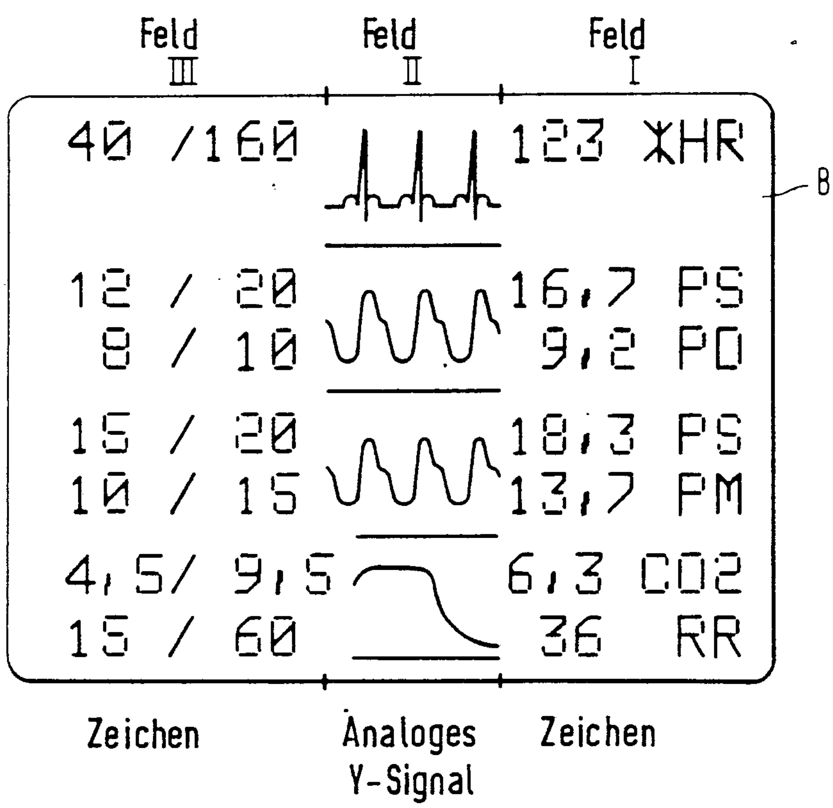
Feld
III
Feld
II
Feld
I
40 /160
123 HR
B
12 / 20
8 / 10
16,7 PS
9,2 PD
15 / 20
10 / 15
18,3 PS
13,7 PM
4,5/ 9,5
15 / 60
6,3 CO2
36 RR
Zeichen
Analoges
Y-Signal
Zeichen

FIG. 3

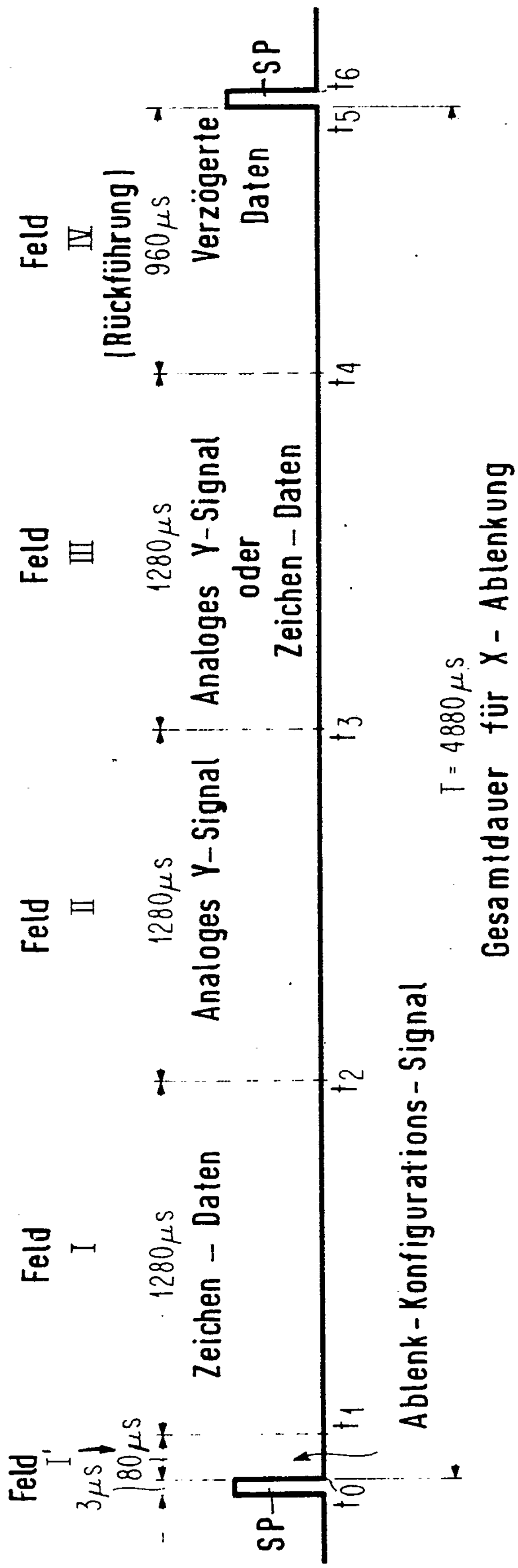
SP
Feld I'
3 μs
80 μs
t0
t1
Feld I
1280 μs
Zeichen – Daten
t2
Feld II
1280 μs
Analoges Y–Signal
t3
Feld III
1280 μs
Analoges Y–Signal
oder
Zeichen – Daten
t4
Feld IV
(Rückführung)
960 μs
Verzögerte
Daten
t5
t6
SP
Ablenk-Konfigurations-Signal
T = 4880 μs
Gesamtdauer für X - Ablenkung

FIG. 4

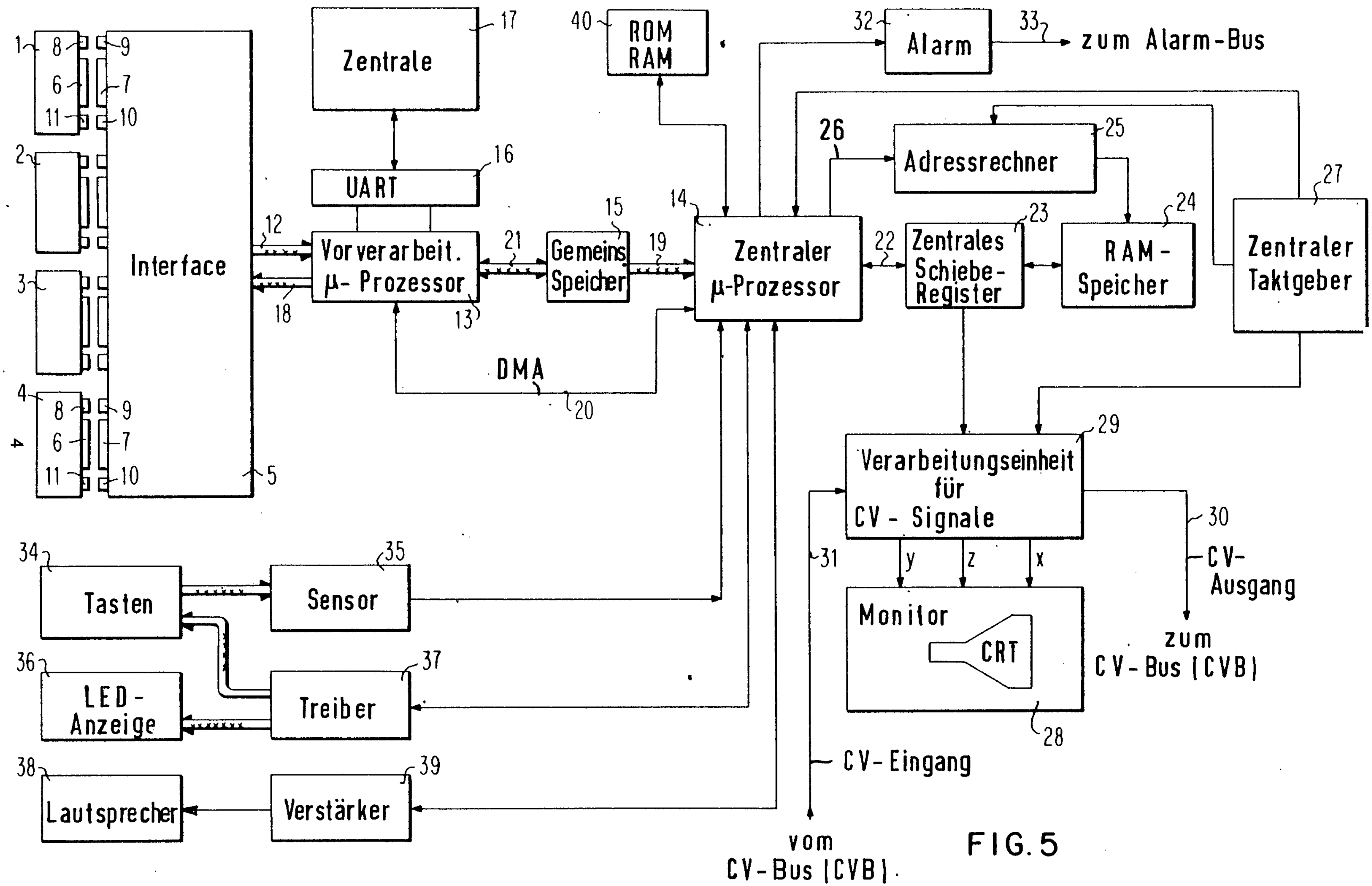

zum Alarm-Bus
Alarm
ROM RAM
Zentrale
UART
Vorverarbeit. μ- Prozessor
Gemeins Speicher
DMA
Zentraler μ-Prozessor
Adressrechner
Zentrales Schiebe-Register
RAM-Speicher
Zentraler Taktgeber
Verarbeitungseinheit für CV - Signale
Monitor
CRT
y
z
x
CV-Ausgang
zum CV-Bus (CVB)
CV-Eingang
vom CV-Bus (CVB)
Interface
Tasten
Sensor
LED-Anzeige
Treiber
Lautsprecher
Verstärker
1
2
3
4
5
6
7
8
9
10
11
12
13
14
15
16
17
18
19
20
21
22
23
24
25
26
27
28
29
30
31
32
33
34
35
36
37
38
39
40


FIG.5

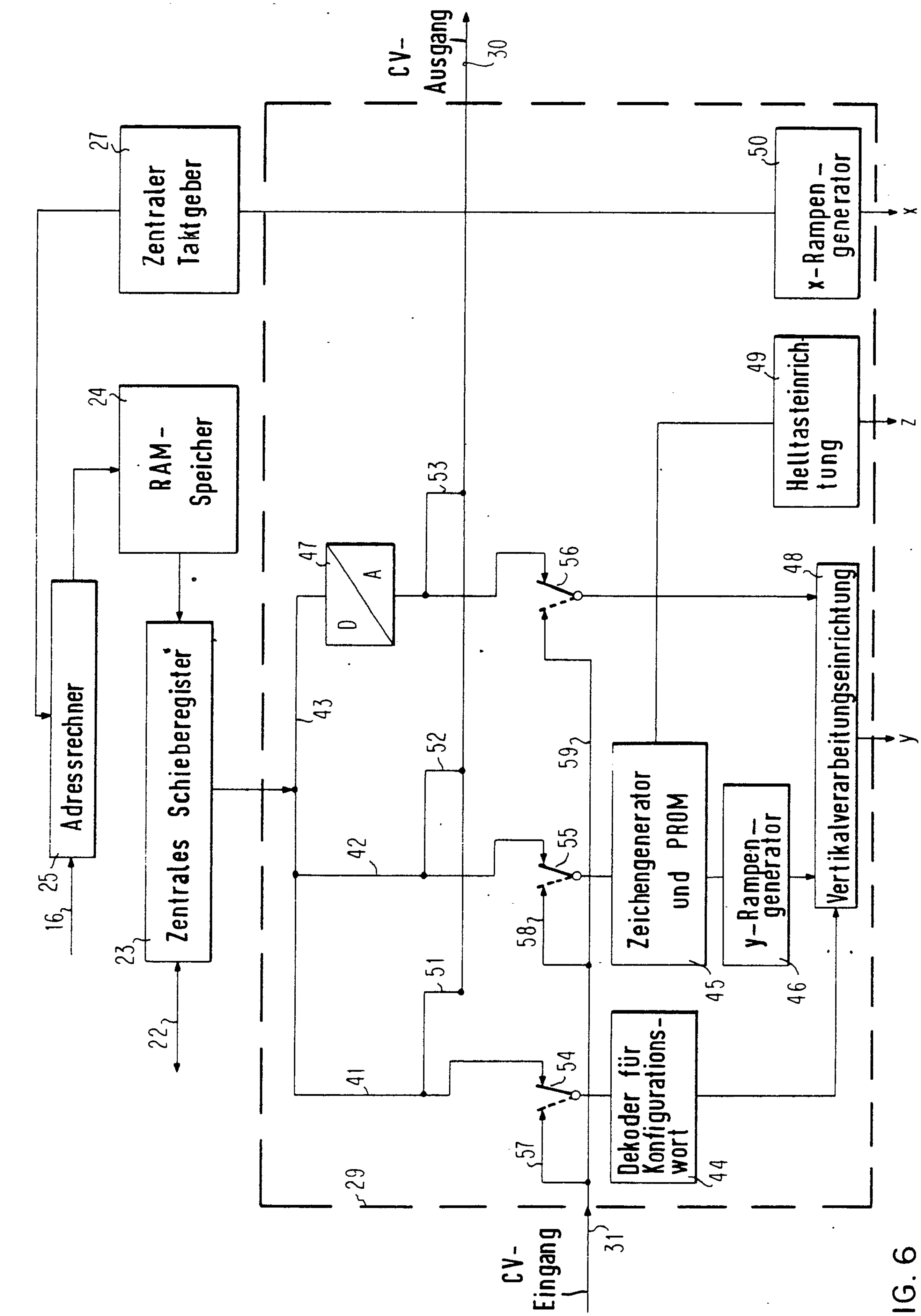
CV-Ausgang
30
Zentraler Taktgeber
27
RAM-Speicher
24
Adressrechner
25
16
Zentrales Schieberegister
23
22
x-Rampengenerator
50
x
Helltasteinrichtung
49
z
53
47
D
A
56
48
Vertikalverarbeitungseinrichtung
y
43
52
59
Zeichengenerator und PROM
45
y-Rampengenerator
46
42
55
58
51
41
54
57
Dekoder für Konfigurationswort
44
29
CV-Eingang
31

FIG. 6

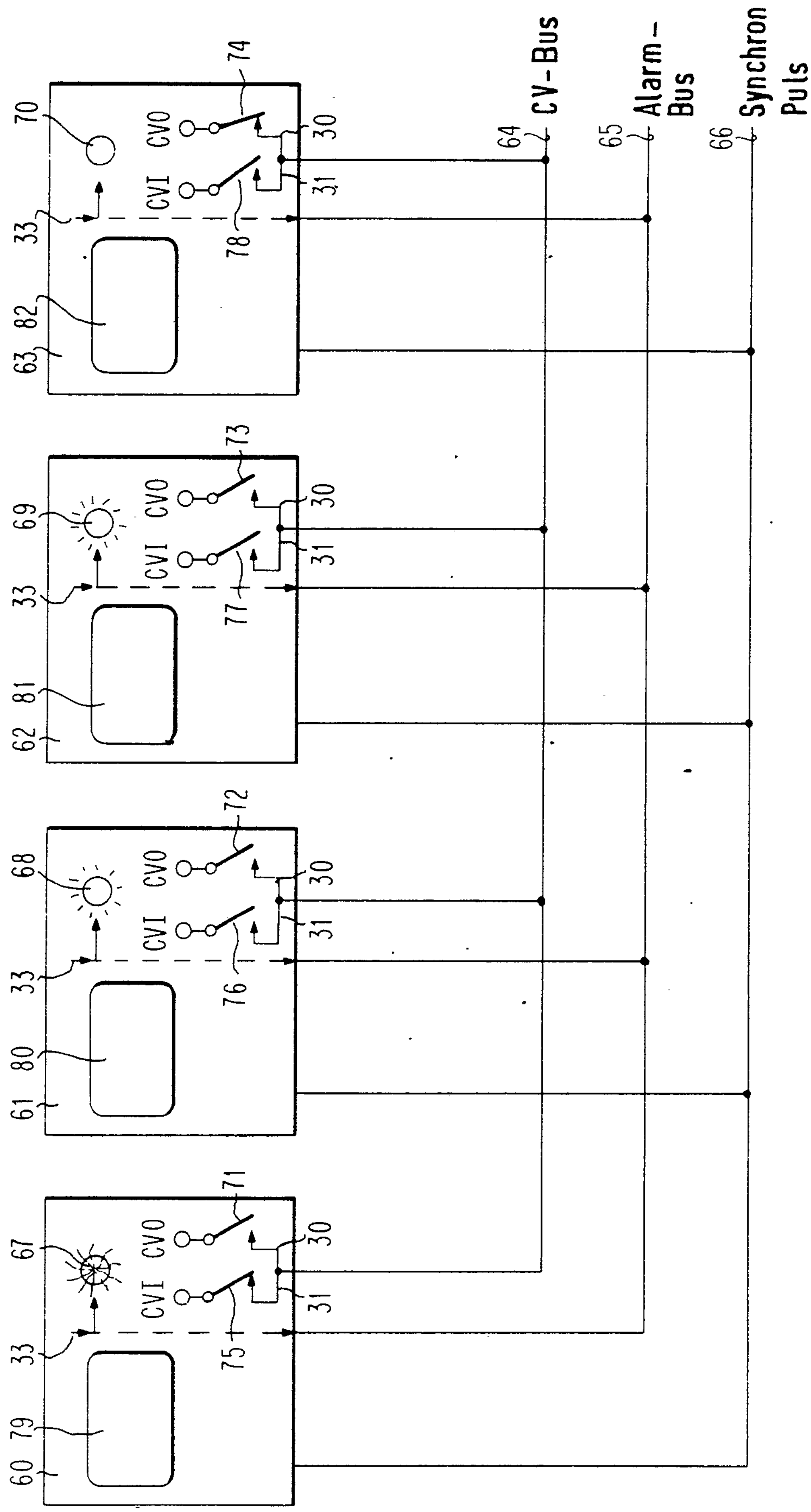

CV-Bus
Alarm-Bus
Synchron Puls
64
65
66
CVI
CVO
30
31
33
60
61
62
63
67
68
69
70
71
72
73
74
75
76
77
78
79
80
81
82


FIG. 7

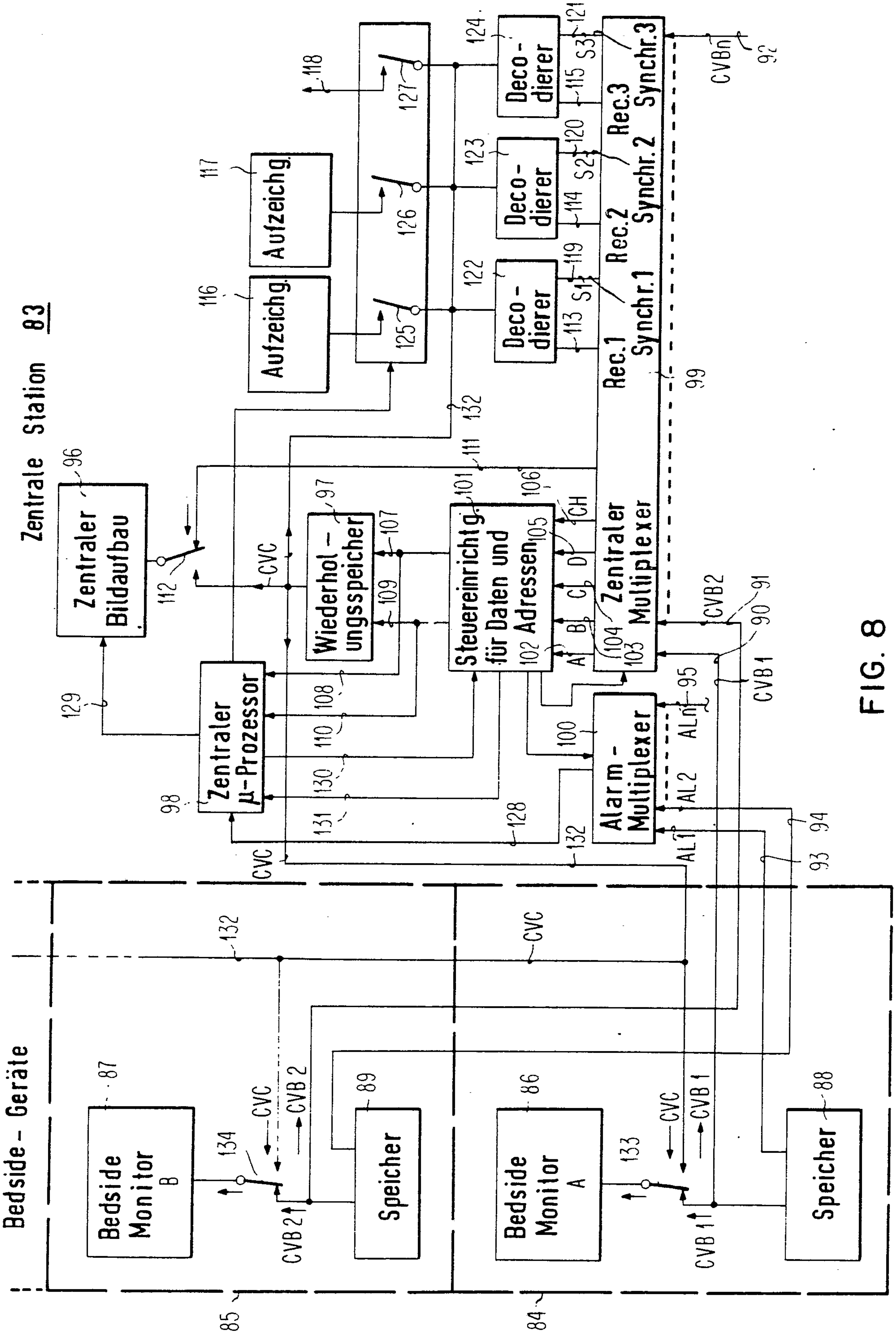
Bedside - Geräte
Bedside Monitor B
Bedside Monitor A
Speicher
Speicher
CVC
CVB 2
CVB 1
CVB 2
CVB 1
Zentrale Station 83
Zentraler Bildaufbau
Zentraler µ-Prozessor
Wiederholungsspeicher
Steuereinrichtg. für Daten und Adressen
Zentraler Multiplexer
Alarm-Multiplexer
Aufzeichg.
Aufzeichg.
Decodierer
Decodierer
Decodierer
Rec.1 Synchr.1
Rec.2 Synchr.2
Rec.3 Synchr.3
CVBn
AL1
AL2
ALn
CH
A
B
C
D
S1
S2
S3
84
85
86
87
88
89
90
91
92
93
94
95
96
97
98
99
100
101
102
103
104
105
106
107
108
109
110
111
112
113
114
115
116
117
118
119
120
121
122
123
124
125
126
127
128
129
130
131
132
133
134

FIG. 8

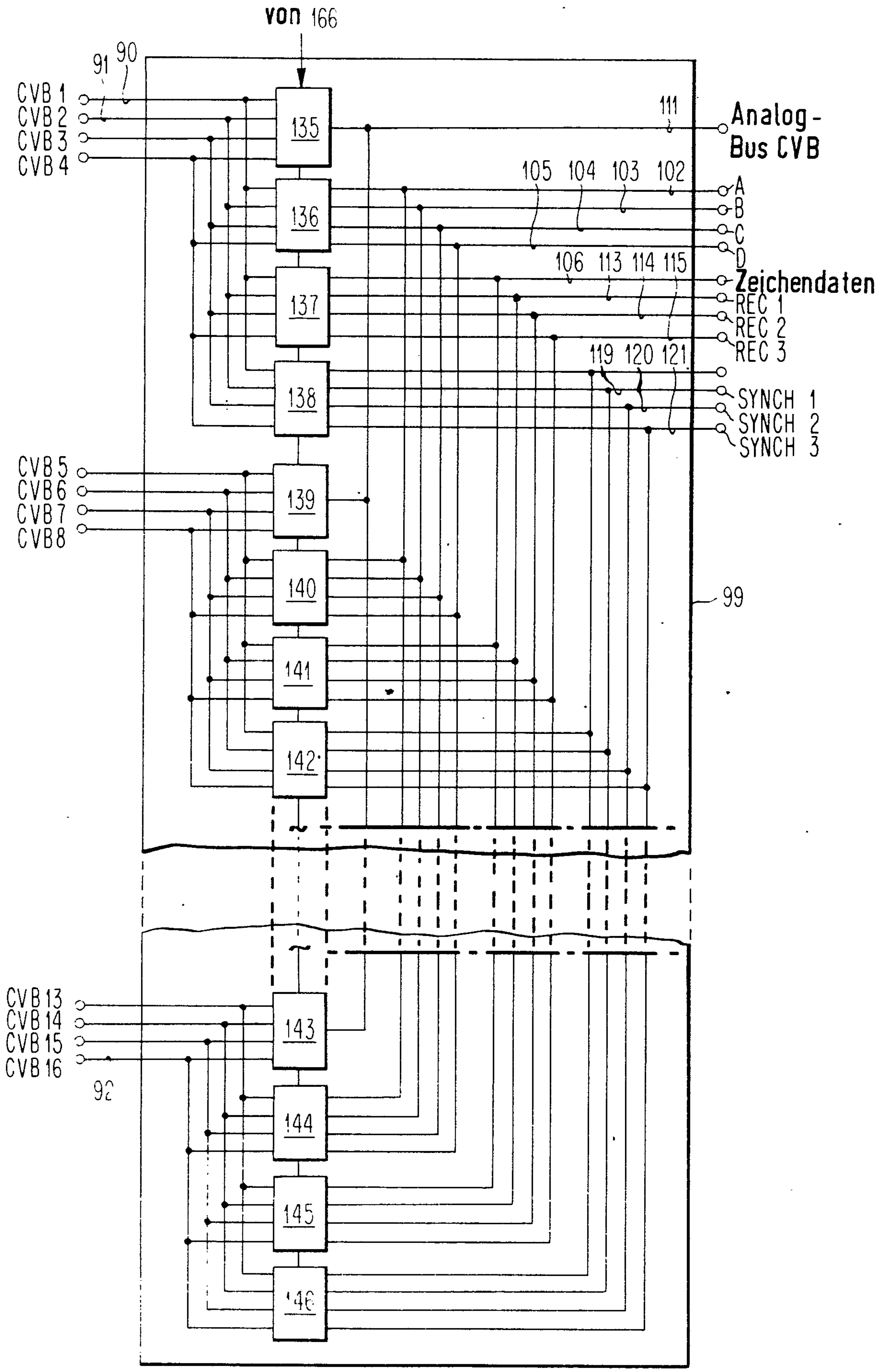
von 166
91
90
CVB 1
CVB 2
CVB 3
CVB 4
135
136
137
138
111
Analog-
Bus CVB
105 104 103 102
A
B
C
D
106 113 114 115
Zeichendaten
REC 1
REC 2
REC 3
119 120 121
SYNCH 1
SYNCH 2
SYNCH 3
CVB 5
CVB 6
CVB 7
CVB 8
139
140
141
142
99
CVB 13
CVB 14
CVB 15
CVB 16
92
143
144
145
146

FIG. 9

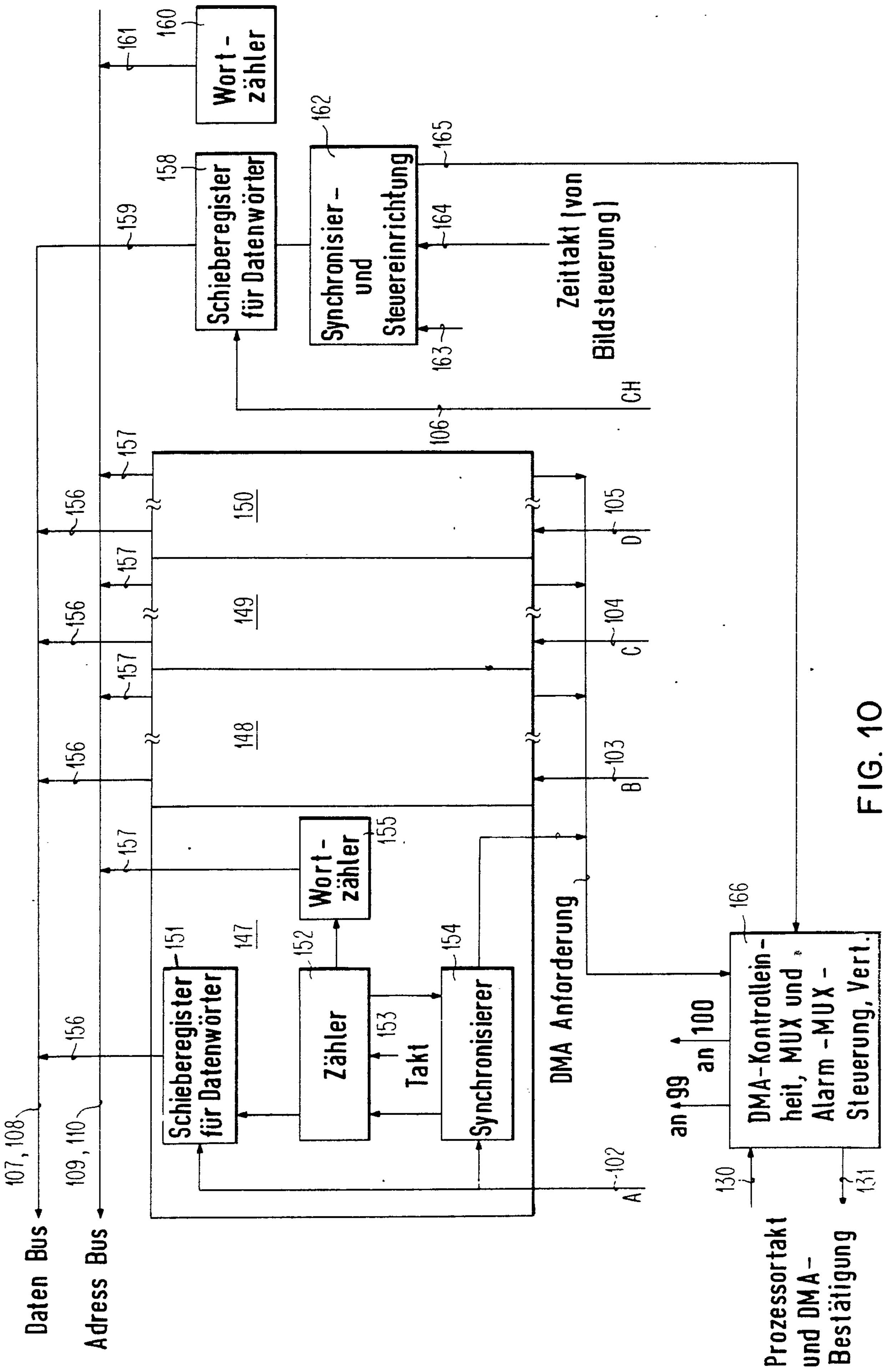

Wort-zähler
160
161
Schieberegister für Datenwörter
158
159
Synchronisier- und Steuereinrichtung
162
163
164
165
Zeittakt (von Bildsteuerung)
CH
106
157
156
150
149
148
147
105
D
104
C
103
B
102
A
Wort-zähler
155
Schieberegister für Datenwörter
151
Zähler
152
153
Takt
Synchronisierer
154
DMA Anforderung
DMA-Kontrolleinheit, MUX und Alarm-MUX-Steuerung, Vert.
166
an 99
an 100
130
131
Prozessortakt und DMA-Bestätigung
Daten Bus
Adress Bus
107, 108
109, 110


FIG. 10

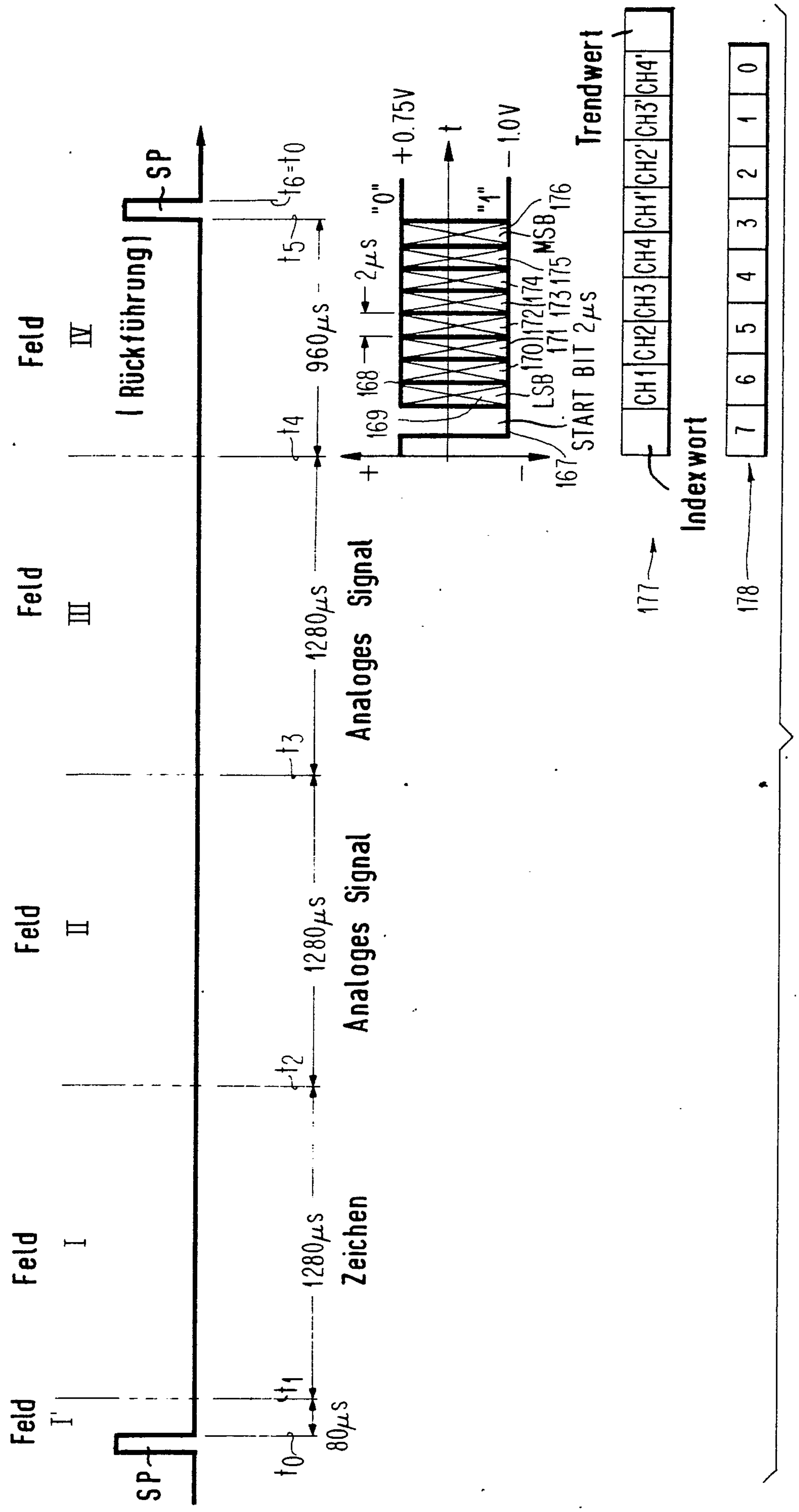
SP
Feld I'
Feld I
Feld II
Feld III
Feld IV
(Rückführung)
SP
t0
t1
t2
t3
t4
t5
t6=t0
80µs
1280µs
1280µs
1280µs
960µs
Zeichen
Analoges Signal
Analoges Signal
+0.75V
-1.0V
"0"
"1"
t
2µs
+
-
167
168
169
170
171
172
173
174
175
176
LSB
MSB
START BIT 2µs
CH1
CH2
CH3
CH4
CH1'
CH2'
CH3'
CH4'
Trendwert
Indexwort
177
178
7
6
5
4
3
2
1
0

FIG. 11

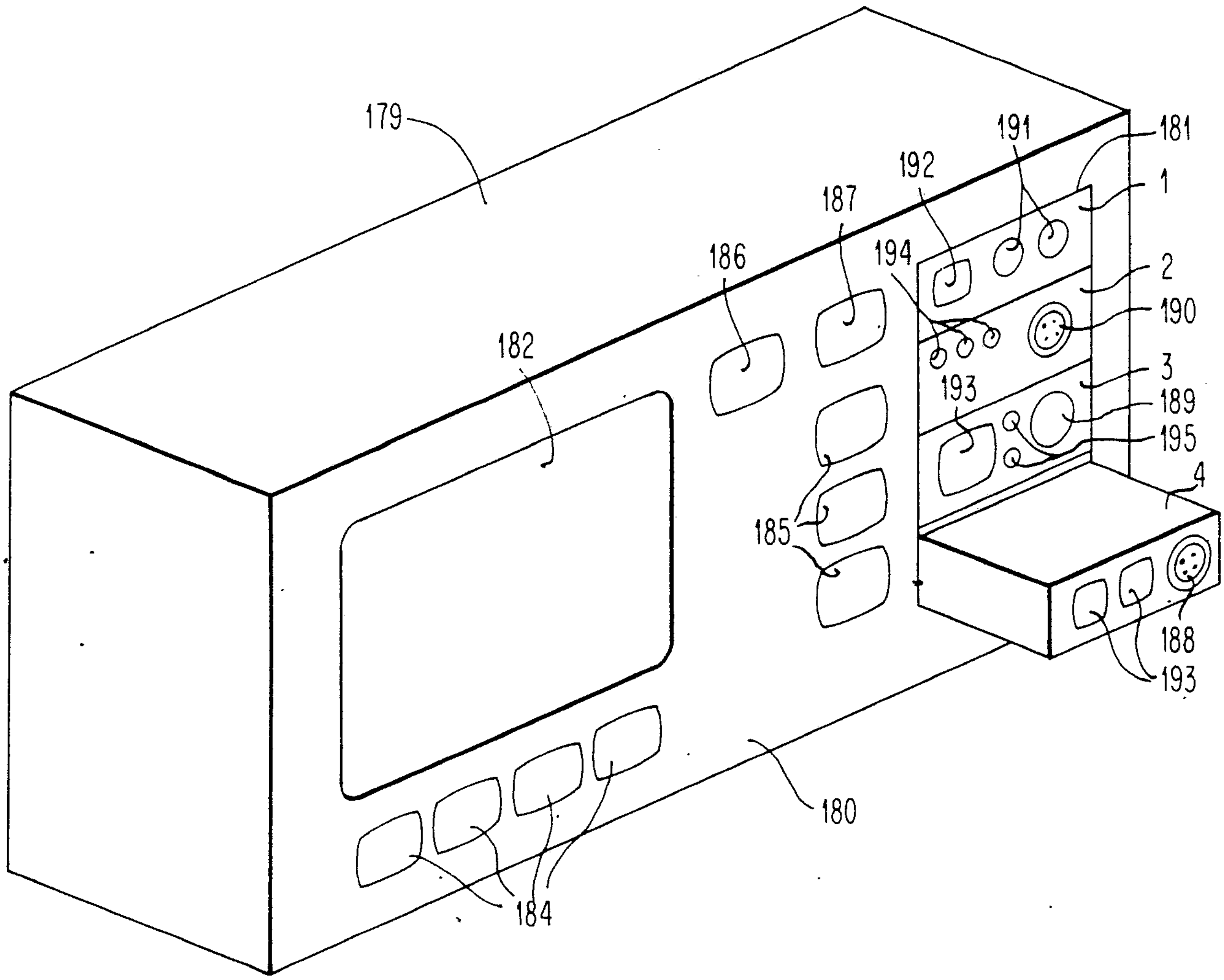
179
192
191
181
1
187
186
194
2
190
182
3
193
189
195
4
185
188
193
180
184

FIG. 12

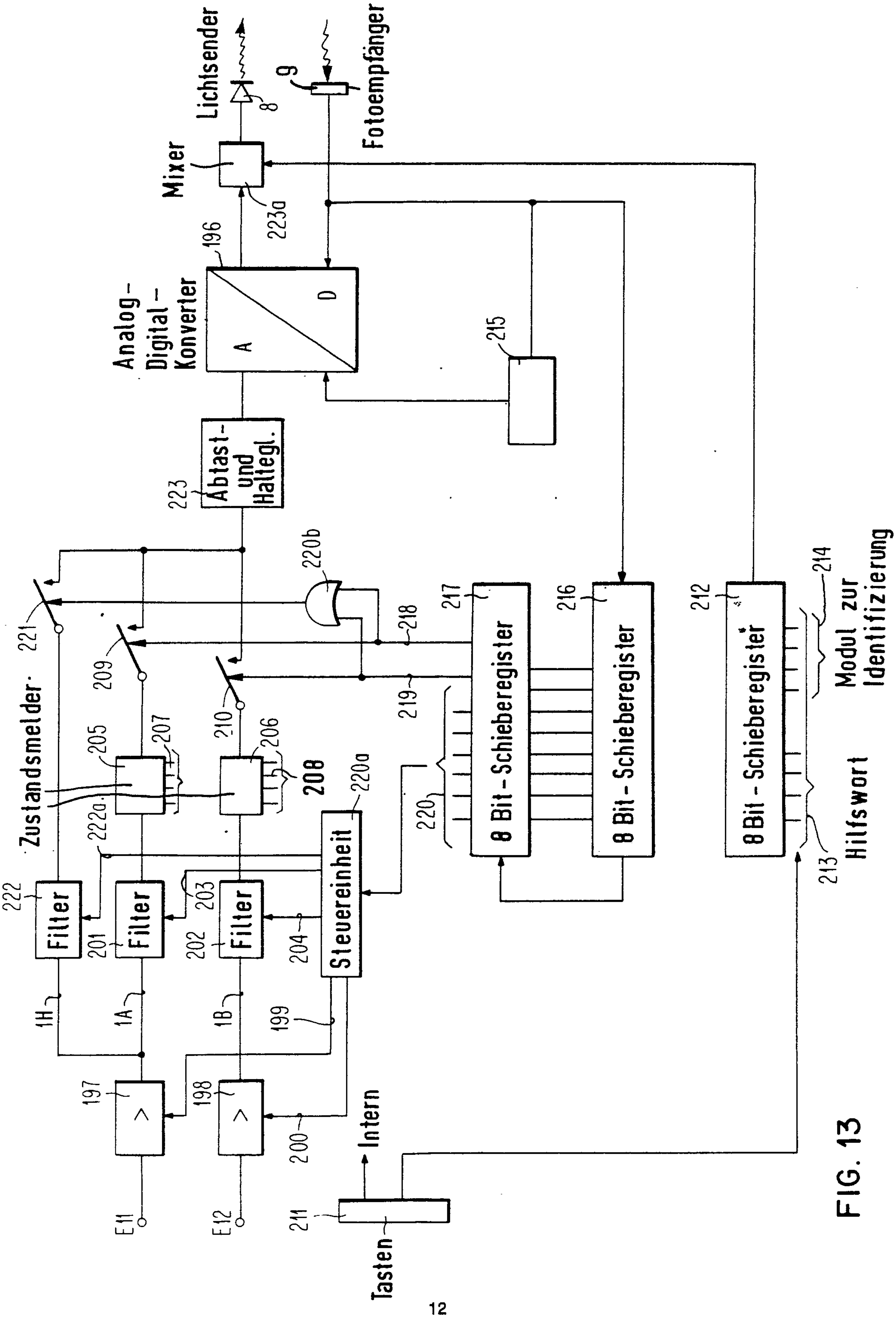
Lichtsender
Fotoempfänger
Mixer
Analog-
Digital-
Konverter
A
D
Abtast-
und
Haltegl.
Zustandsmelder
Filter
Filter
Filter
Steuereinheit
8 Bit - Schieberegister
8 Bit - Schieberegister
8 Bit - Schieberegister
Modul zur
Identifizierung
Hilfswort
Intern
Tasten
E11
E12
1H
1A
1B
8
9
196
197
198
199
200
201
202
203
204
205
206
207
208
209
210
211
212
213
214
215
216
217
218
219
220
220a
220b
221
222
222a
223
223a

FIG. 13

FIG. 14

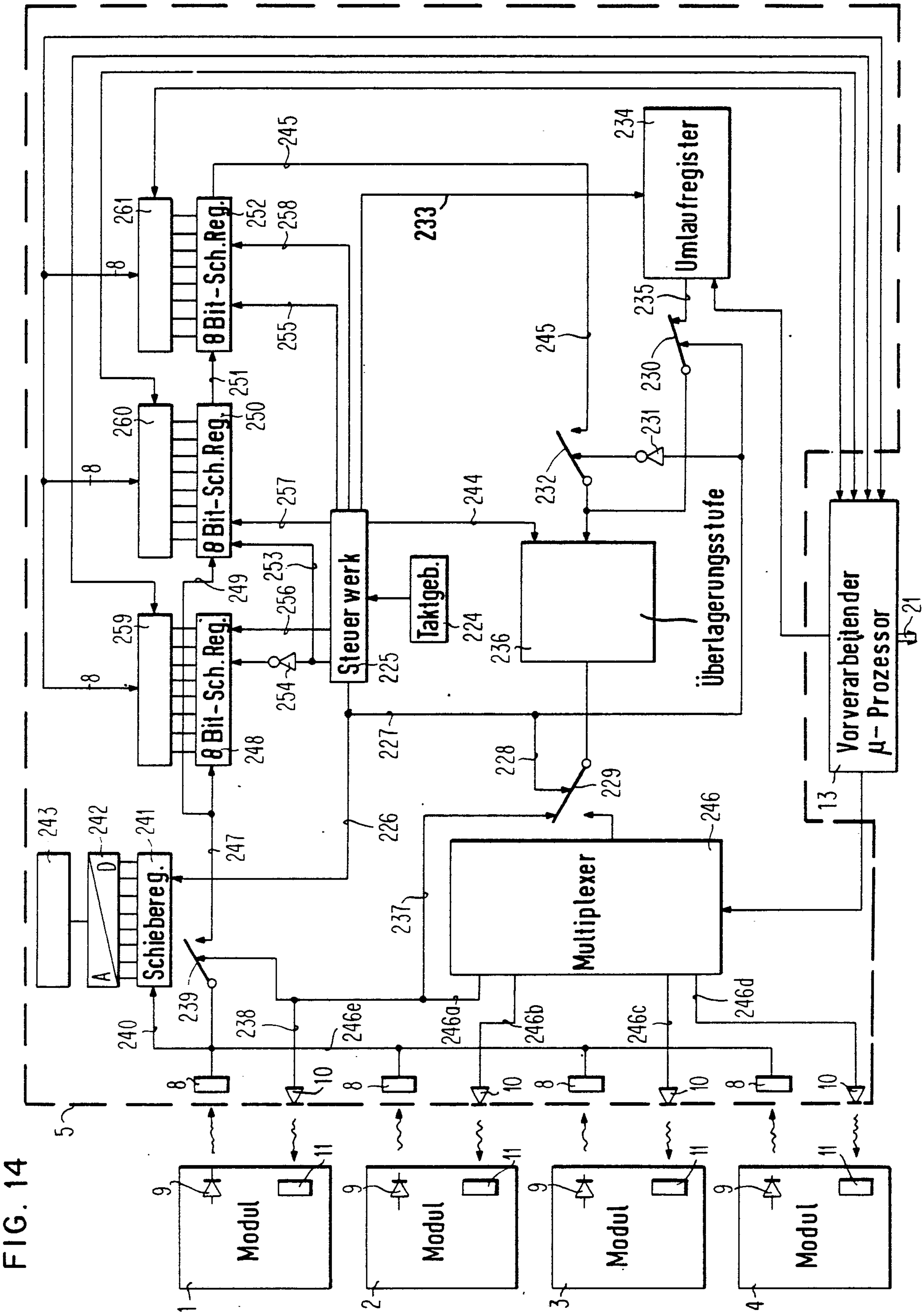
Umlaufregister
8 Bit-Sch.Reg.
8 Bit-Sch.Reg.
8 Bit-Sch.Reg.
Steuer werk
Taktgeb.
Überlagerungsstufe
Schiebereg.
A
D
Multiplexer
Vorverarbeitender µ-Prozessor
Modul
Modul
Modul
Modul

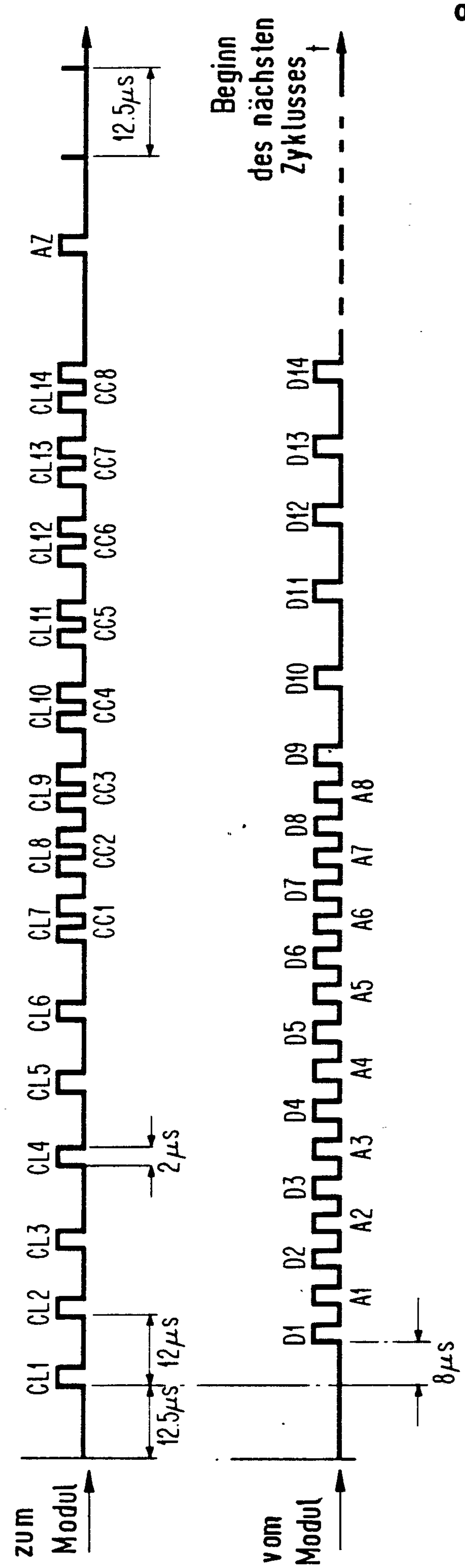
zum Modul
vom Modul
12.5μs
12μs
2μs
8μs
CL1 CL2 CL3 CL4 CL5 CL6 CL7 CL8 CL9 CL10 CL11 CL12 CL13 CL14
CC1 CC2 CC3 CC4 CC5 CC6 CC7 CC8
AZ
D1 D2 D3 D4 D5 D6 D7 D8 D9 D10 D11 D12 D13 D14
A1 A2 A3 A4 A5 A6 A7 A8
Beginn des nächsten Zyklusses

FIG. 15

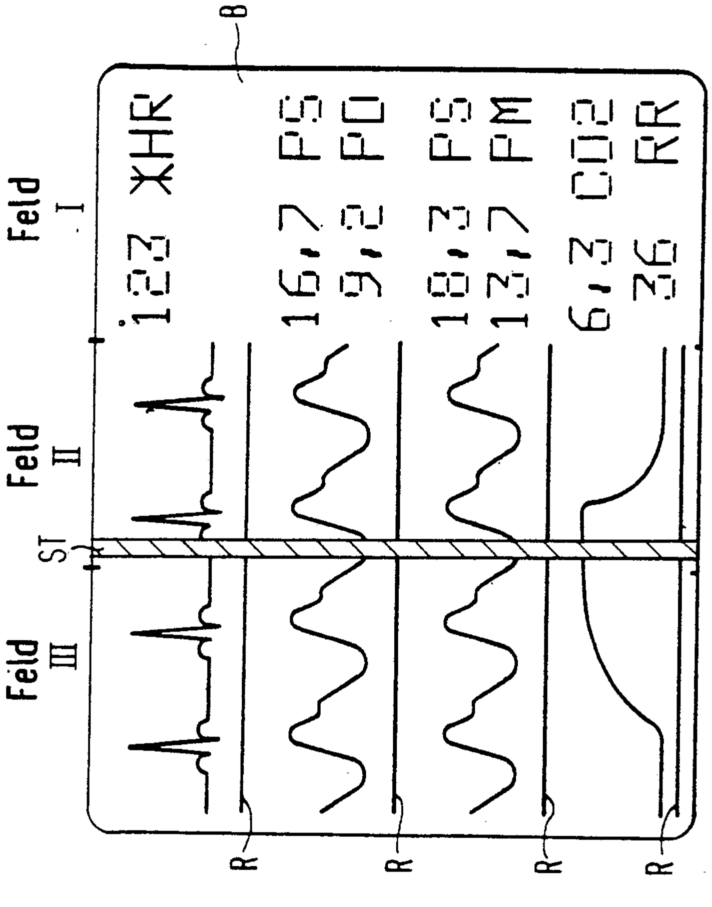
Feld III
ST
Feld II
Feld I
B
R
123 HR
16,7 PS
9,2 PD
18,3 PS
13,7 PM
6,3 CO2
36 RR

FIG. 17

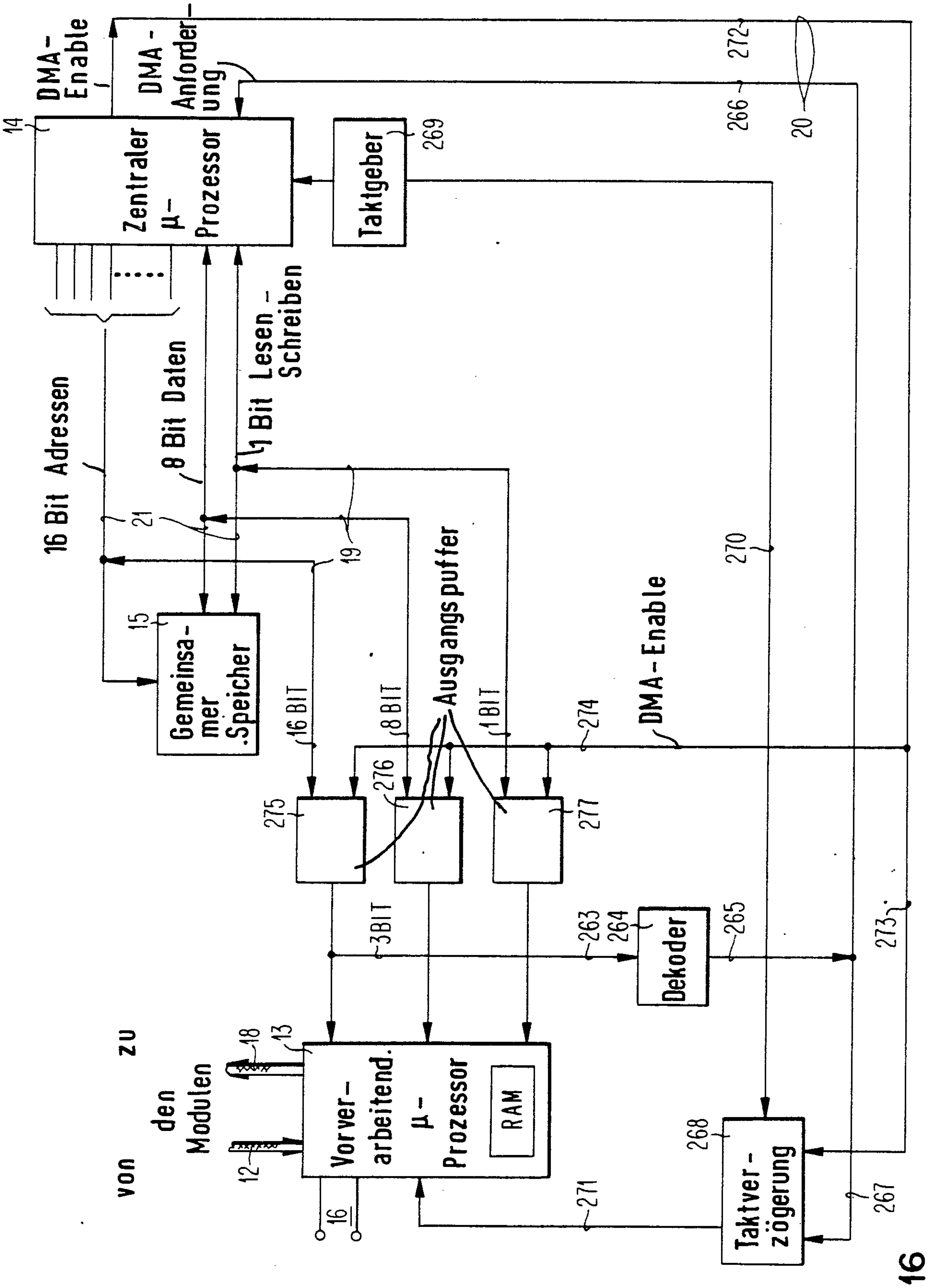
DMA-Enable
DMA-Anforderung
14
Zentraler µ-Prozessor
Taktgeber
269
272
266
20
16 Bit Adressen
8 Bit Daten
1 Bit Lesen-Schreiben
21
19
15
Gemeinsamer Speicher
Ausgangspuffer
16 BIT
8 BIT
1 BIT
274
DMA-Enable
270
275
276
277
3BIT
263
264
Dekoder
265
273
von den Modulen zu
18
13
12
Vorverarbeitend. µ-Prozessor
RAM
16
271
268
Taktverzögerung
267

FIG. 16

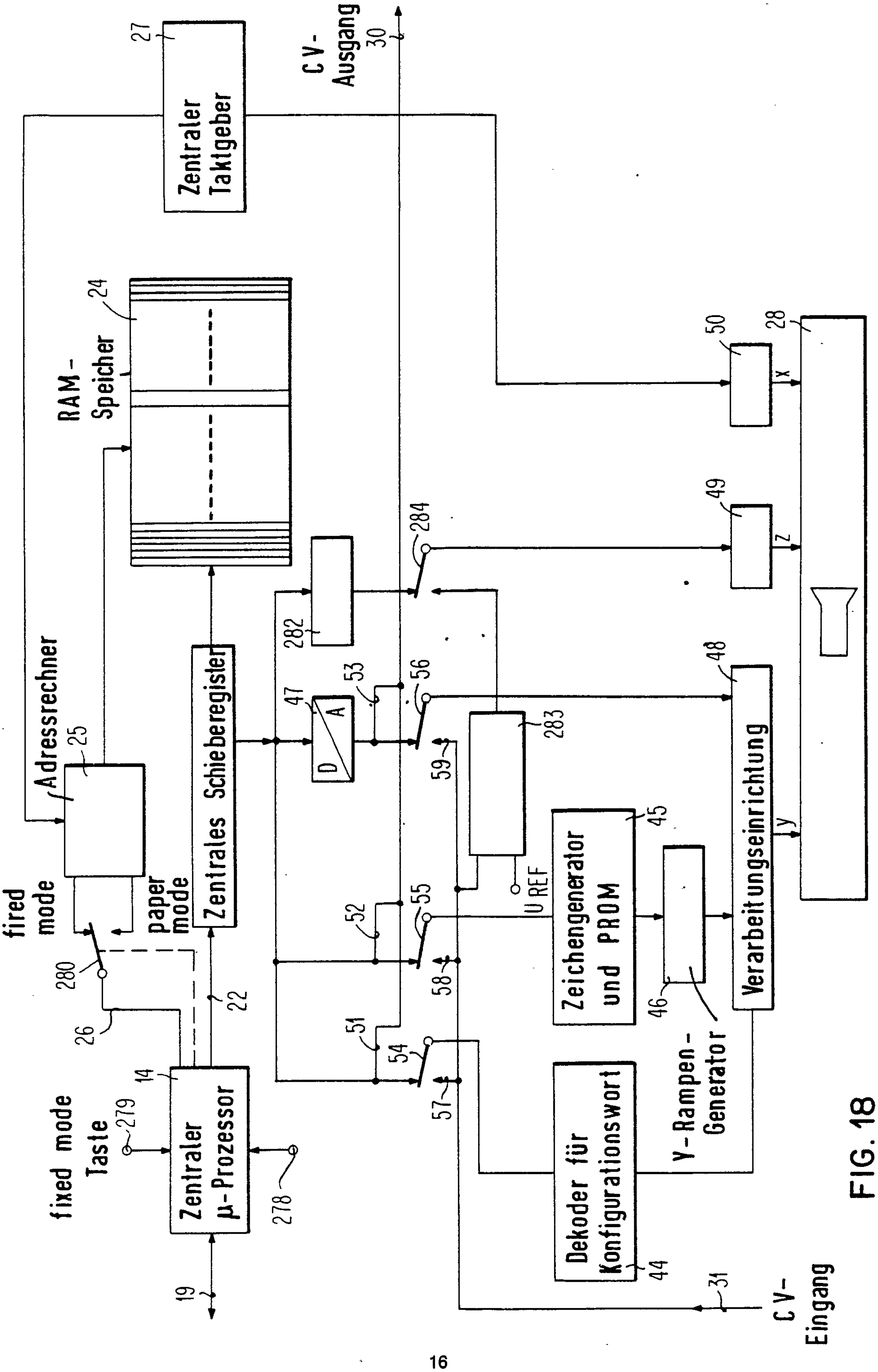
Zentraler Taktgeber
27
CV-Ausgang
30
RAM-Speicher
24
Adressrechner
25
fired mode
paper mode
280
26
22
14
Zentrales Schieberegister
fixed mode Taste
279
Zentraler µ-Prozessor
278
19
D
A
47
53
56
59
282
284
283
U REF
52
55
58
51
54
57
Zeichengenerator und PROM
45
46
Y-Rampen-Generator
Verarbeitungseinrichtung
48
49
z
50
x
y
28
Dekoder für Konfigurationswort
44
31
CV-Eingang

FIG. 18